# EUROPEAN PATENT APPLICATION

(11) **EP 4 438 119 A2**
(43) Date of publication of application: **02.10.2024**
(21) Application number: 24170593.8
(22) Date of filing: 14.02.2022
(51) Int. Cl.: A61P 25/28

(54) **COMPOUND FOR TREATMENT OF COGNITIVE DISORDERS**

(30) Priority: 26.02.2021 EP 21159595
(62) Divisional of application: 22705540.7
(71) Applicant: Syndesi Therapeutics SA, 1348 Louvain-la-Neuve (BE)
(72) Inventor: SAVIDGE, Jonathan, Oxfordshire, OX14 1AZ (GB); KEMP, John, 4059 Basel (CH); CESURA, Andrea, 1299 Crans (CH)
(74) Representative: J A Kemp LLP

(57) **Abstract**

The invention relates to administration at particular dosages of a compound which is a substituted 2-oxo-1-pyrrolidinyl triazole of formula (I) or an isomer or a pharmaceutically acceptable salt thereof.

## Description

### FIELD OF THE INVENTION

The invention relates to compounds and pharmaceutical compositions for use in treatment of cognitive disorders.

### BACKGROUND

Cognitive disorders, i.e. impairments of memory and learning processes, have a significant detrimental effect on the quality of life of patients. Clinically recognized cognitive disorders vary from mild cognitive impairment through to dementia of varying severity. Cognitive disorders may also be associated with many other diseases or disorders such as schizophrenia, depression and Parkinson's disease.

Mild cognitive impairment ("MCI") is believed to be a transition stage between the cognitive changes of normal aging and the more serious problems caused by Alzheimer's disease. Dementia is a clinically recognized broad-spectrum syndrome entailing progressive loss of cognitive capabilities. Dementia can be one of many symptoms of various neurological diseases or the main abnormality associated with the disease, as is the case in Alzheimer's disease. The most common causes of dementia include Alzheimer's disease, Lewy-bodies disease, front-temporal lobe degeneration, Pick's disease, vascular narrowing or blockage in the brain (i.e. vascular dementia also known as multi-infarct dementia), Huntington's disease, Parkinson's disease, head trauma, HIV infection or Down's syndrome.

Alzheimer's disease (AD) is a progressive degenerative disease of the brain primarily associated with aging. AD is one of several disorders that cause the gradual loss of brain cells and is one of the leading causes of dementia. Clinical presentation of AD is characterized by loss of memory, cognition, reasoning, judgment, and orientation. Mild cognitive impairment is often the first identified stage of AD. As the disease progresses, motor, sensory, and linguistic abilities also are affected until there is global impairment of multiple cognitive functions. These cognitive losses occur gradually, but typically lead to severe impairment, and the disease leads eventually to death in around three to twenty years. Currently there are only a few medications that have been shown to afford at most a modest, mostly transient benefit to the patients suffering from cognitive impairment. Cholinesterase inhibitors (anticholinesterases), such as donepezil (Aricept^{®}), galanthamine (Razadyne^{®}, Razadyne ER^{®}, Reminyl^{®}, Nivalin^{®}) and rivastigmine tartrate (Exelon^{®}) have been shown to be efficacious in mild to moderate Alzheimer's disease dementia. Exelon^{®} has recently been approved for the treatment of mild to moderate dementia associated with Parkinson's disease. Memantine, a NMDA receptor antagonist, is the first approved Alzheimer's disease medication acting on the glutamatergic system (Axura^{®}, Akatinol^{®}, Namenda^{®}, Ebixa^{®}). These drugs have limited proven efficacy and considerable side effects, which in some cases lead to discontinuation of the therapy. With the increase in the life span and general aging of the population there is a need to develop drugs which could delay or alleviate the decline in cognitive function in aging patients.

Cognitive impairment associated with schizophrenia (CIAS) is an intrinsic part of the illness, affecting the majority of the patients, and often pre-dates its onset. It affects a wide range of cognitive functions, particularly memory, attention, motor skills, executive function and social cognition following dysregulation of several neurotransmitter systems.

Levetiracetam or (S)-(-)-alpha-ethyl-2-oxo-1-pyrrolidine acetamide, is a levorotatory compound, disclosed in the European patent EP162036 as being a protective agent for the treatment and the prevention of hypoxic and ischemic type damages of the central nervous system. Levetiracetam has been approved, and is marketed as Keppra^{®}, for the treatment of various forms of epilepsy in many countries including the European Union and the United States. Epilepsy is a therapeutic indication for which that the dextrorotatory enantiomer of levetiracetam [(R)-(+)-alpha-ethyl-2-oxo-1-pyrrolidine acetamide] completely lacks activity (Gower et al., Eur. J. Pharmacol. 222, 193-203 (1992)). It has been repeatedly reported however that levetiracetam has no impact on the cognitive function both in animals as well as in humans (Lamberty et al, Epilepsy & Behavior 1 , 333- 342 (2000); Klitgaard et al. Epilepsy Research 50, 55-65 (2002); Shannon H & Love, P. Epilepsy & Behavior 7, 620-628 (2005); Higgins et al. Psychopharmacology 207, 513-527 (2010)). The chemical analog of levetiracetam, brivaracetam [(2S)-2-[(4R)-2-oxo-4-propyltetrahydro-1H-pyrrol-1-yl] butanamide] has also been approved for use in humans to treat epilepsy and is marketed as Briviact.

SV2A is involved in vesicle trafficking and exocytosis, specifically the priming of docked vesicles to make them available for calcium-dependent fusion and, thereby, neurotransmitter release (Chang & Sudhof, J. Neurosci 2009 29 (4) 883-887. Both levetiracetam and brivaracetam are SV2A inhibitors, inhibiting synaptic function by reducing pre-synaptic vesicle fusion and neurotransmitter release. Levetiracetam and brivaracetam act as anticonvulsants. Brivaracetam binds to SV2A with a higher affinity than levetiracetam.

Kaminski et al. Targeting SV2A for Discovery of Antiepileptic Drugs, Jasper's Basic Mechanisms of the Epilepsies, 4th edition, 2012, describes the relationship between SV2A occupancy and seizure occurrence. In studies in audiogenic mice, it was found that levetiracetam doses providing maximum SV2A occupancy provided the best protection against clonic and tonic seizures. These studies also revealed that levetiracetam needed to occupy nearly 90% of SV2A in order to protect against clonic seizure in mice i.e. that a threshold SV2A occupancy needed to be reached to protect against seizures. Predictions for human brains based on clinically active daily dosing showed that SV2A occupancy varies from 80 to 93% at Cₘᵢₙ and Cₘₐₓ for patients receiving a 1 g daily dose of levetiracetam, and from 92 to 98% for patients receiving a 3 g daily dose. Finnema et al., A single-center, open-label positron emission tomography study to evaluate brivaracetam and levetiracetam synaptic vesicle glycoprotein 2A binding in healthy volunteers, Epilepsia 2019; 60, 958-967, showed that after oral administration of 100 mg for 4 days an occupancy of 82-87% and 76-82% was reached at steady state at peak and trough respectively. These studies confirmed that high SV2A occupancy is needed for levetiracetam and brivaracetam to provide adequate protection against seizures.

Dysfunction of SV2A may also be involved in Alzheimer's disease and other types of cognitive impairment (Löscher et al., CNS Drugs 2016, 30: pages 1055-1077). High-affinity SV2A ligands have been discovered which are devoid of anti-seizure activity, but provide pro-cognitive effects.

### SUMMARY OF THE INVENTION

The present invention provides compounds that bind with high affinity to SV2A and compositions comprising such compounds for use in treating a cognitive disorder in a subject. These compounds increase neurotransmitter release presynaptically i.e. are positive modulators of SV2A, rather than inhibitors. In particular, the invention relates to treatments which involve administering these compounds and compositions in an amount which will result in levels of SV2A occupancy useful for imparting pro-cognitive effects. The inventors have surprisingly discovered that low doses of the compounds described herein, corresponding to partial SV2A occupancy, modulate brain activity in specific areas associated with a potential beneficial effect on cognitive function while being safe and well tolerated without major side effects. These findings open up the possibility of treating cognitive disorders and improving cognitive function by dosing the compounds to achieve a specific range of SV2A occupancy levels.

The invention therefore provides a compound which is a substituted 2-oxo-1-pyrrolidinyl triazole of formula (I) 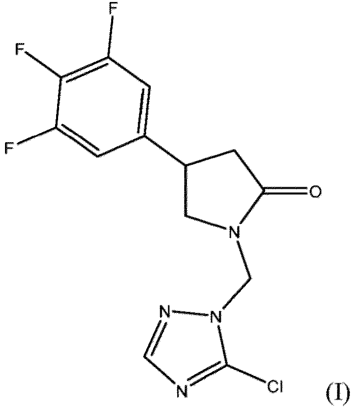 or an isomer or a pharmaceutically acceptable salt thereof, for use in the treatment of a cognitive disorder in a subject, wherein the compound is administered to the subject in an amount of from 0.2 to 5 mg.

The invention also provides a compound which is a substituted 2-oxo-1-pyrrolidinyl triazole of formula (I) 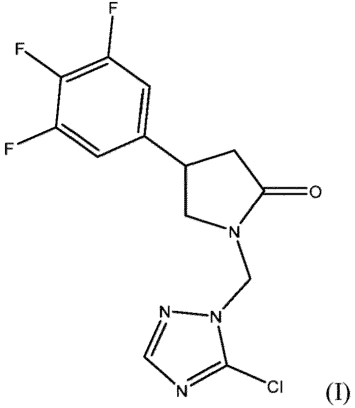 or an isomer or a pharmaceutically acceptable salt thereof, for use in the treatment of a cognitive disorder in a subject, wherein the compound is administered to the subject in an amount that provides a synaptic vesicle glycoprotein 2A (SV2A) occupancy of from 10% to 80% at trough plasma concentrations after once daily dosing for at least 10 days.

The invention also provides a pharmaceutical composition comprising a compound which is a substituted 2-oxo-1-pyrrolidinyl triazole of formula (I) 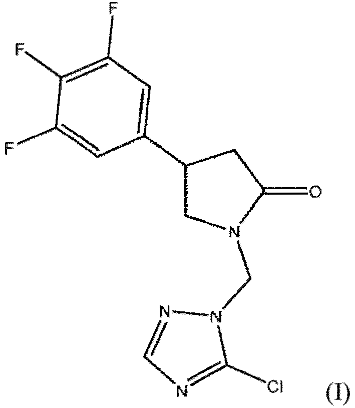 or an isomer or a pharmaceutically acceptable salt thereof, and one or more pharmaceutically acceptable excipients, for use in the treatment of a cognitive disorder in a subject, wherein the pharmaceutical composition is administered to deliver to the subject an amount of the compound as described herein.

The invention also provides a method of treating a cognitive disorder in a subject, the method comprising administering a compound which is a substituted 2-oxo-1-pyrrolidinyl triazole of formula (I) 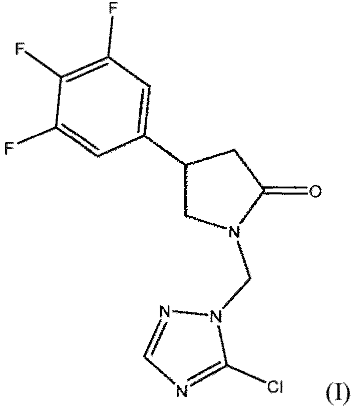 or an isomer or a pharmaceutically acceptable salt thereof to the subject in an amount of from 0.2 to 5 mg.

The invention also provides a method of treating a cognitive disorder in a subject, the method comprising administering a compound which is a substituted 2-oxo-1-pyrrolidinyl triazole of formula (I) 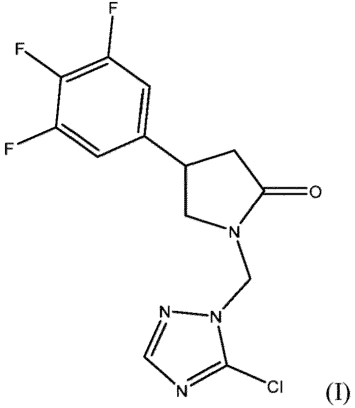 or an isomer or a pharmaceutically acceptable salt thereof to the subject in an amount that provides a synaptic vesicle glycoprotein 2A (SV2A) occupancy of from 10% to 80% at trough level after once daily dosing for at least 10 days.

The invention also provides the use of a compound which is a substituted 2-oxo-1-pyrrolidinyl triazole of formula (I) 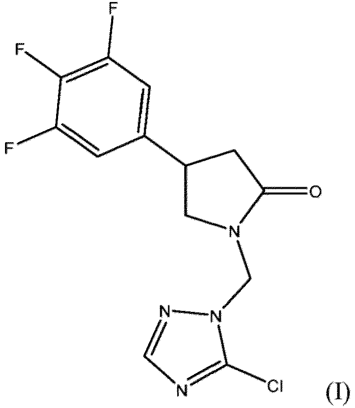 or an isomer or a pharmaceutically acceptable salt thereof, in the manufacture of a medicament for the treatment of a cognitive disorder, wherein the medicament comprises from 0.2 to 5 mg of the compound.

The invention also provides the use of a compound which is a substituted 2-oxo-1-pyrrolidinyl triazole of formula (I) 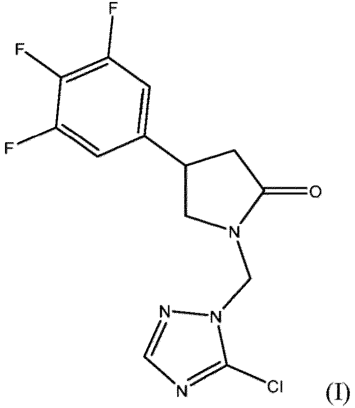 or an isomer or a pharmaceutically acceptable salt thereof, in the manufacture of a medicament for the treatment of a cognitive disorder, wherein the medicament comprises an amount of the compound that provides a synaptic vesicle glycoprotein 2A (SV2A) occupancy of from 10% to 80% at trough level after once daily dosing for at least 10 days.

### BRIEF DESCRIPTION OF THE FIGURES

Figure 1 shows probability maps for brain areas in rats showing significantly dose-dependent changes in BOLD (blood oxygen level dependent) signal with (4R)-1-[(5-chloro-1H-1,2,4-triazol-1-yl)methyl]-4-(3,4,5- trifluorophenyl)pyrrolidin-2-one (free base). The data were generated from Table 1 in example 2.

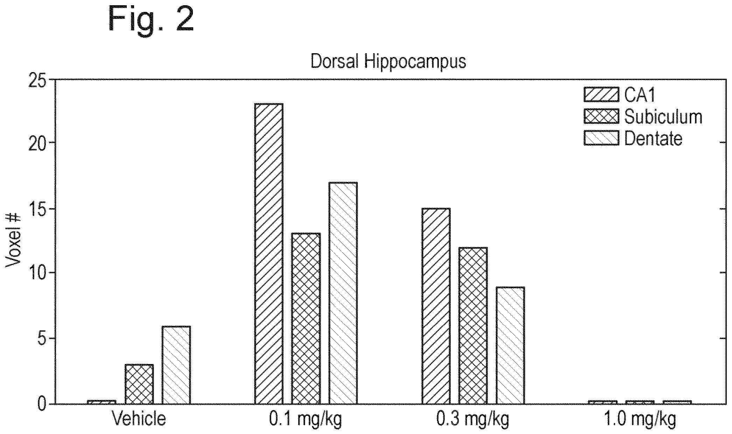

Figure 2 shows a bar graph of the dose-dependent change in volume of activation for the dorsal hippocampus (CA1, subiculum, dentate) in rats in response to the various doses of the compound of example 1.

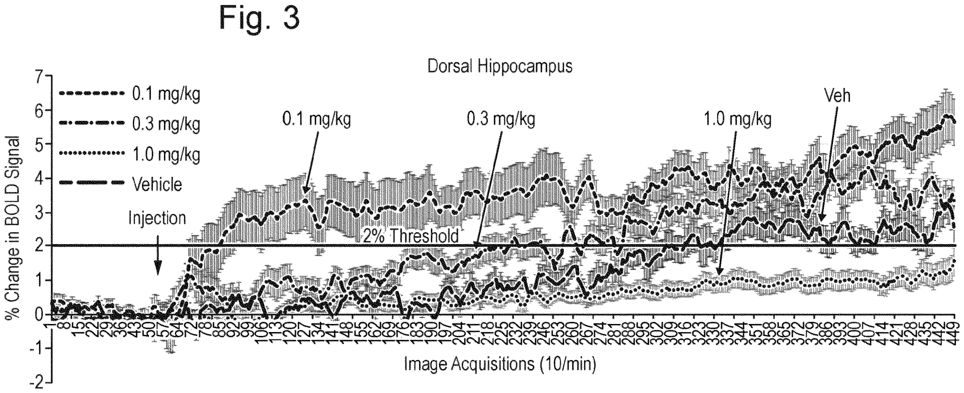

Figure 3 shows the time course for change in BOLD signal in the dorsal hippocampus (all areas combined) in rats following injection of the various doses of the compound of example 1.

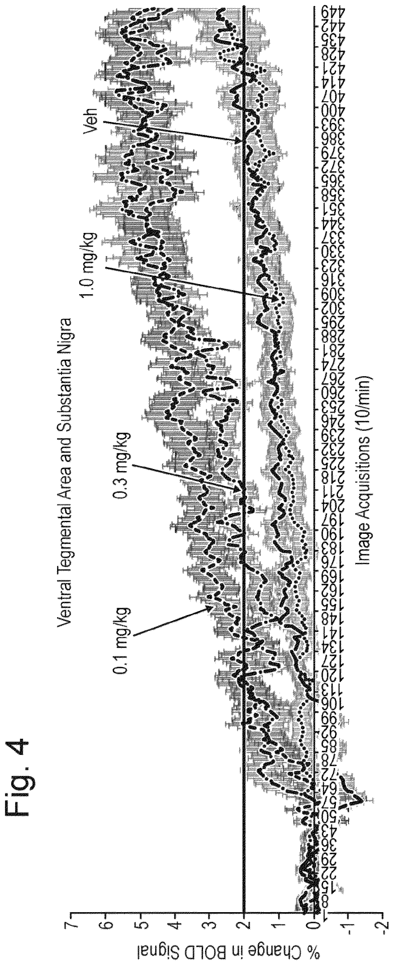

Figure 4 shows the time course for change in BOLD signal in the ventral tegmental area (VTA) and substantia nigra (reticularis and compacta) combined in rats following injection of the various doses of the compound of example 1.
Figure 5 shows a plot of measured plasma concentration in ng/mL vs % SV2A occupancy in humans. Also shown are the 95% confidence intervals and predicted occupancy levels (95%) at varying plasma concentration. EC₅₀ was calculated to be 24.9 ng/mL.
Figure 6 shows calculated of SV2A occupancy based on measured plasma levels of the compound of example 1 in humans. Plasma level data for 5, 10 and 20 mg doses were calculated from experimental data from the dosage study in example 3. The data points for lower doses were extrapolated from the experimental data for the 5mg dose on the basis of the observed linear pharmacokinetics. After day 1, where there were multiple sampling timepoints in a 24 hour period, the graph shows trough SV2A occupancy at trough plasma levels.
Figure 7 shows the plasma levels in humans of the compound of example 1 used as the basis for calculating the SV2A occupancies shown in Figure 6.
Figure 8 shows the degree of spatial match of the different Independent Components (IC) with predefined functional networks in the resting-state function MRI data in example 4.
Figure 9 shows increased connectivity within the DMN using a seed based connectivity analysis with IC12 (DMN) as seed region in the resting-state function MRI data in example 4.
Figure 10 shows increased connectivity of the visual network with the auditory network using a seed based connectivity analysis with IC18 (part of the visual network) as seed region in the resting-state function MRI data in example 4.
Figure 11 shows changes in DMN connectivity in the individual subjects tested with the various doses of the compound in example 4. The beta correlation values represent functional connectivity between a seed and target area (separately for each subject and condition) i.e. the strength of the functional connectivity between two regions. These values are unitless and are shown in the Y-axis of Figure 11.

### DETAILED DESCRIPTION

The invention relates to administration at particular dosages of a compound which is a substituted 2-oxo-1-pyrrolidinyl triazole of formula (I) 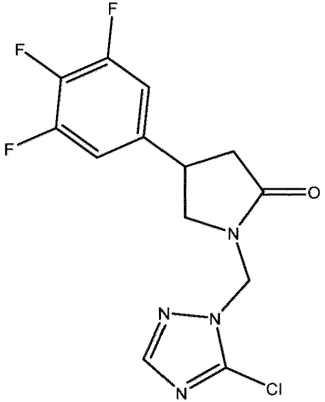 or an isomer or a pharmaceutically acceptable salt thereof.

In the context of the present application, the term "isomer" refers to any geometric, optical, enantiomeric, diastereomeric, epimeric, atropic, stereoisomeric, tautomeric, conformational, or anomeric form of the compound. Preferably, the term isomer refers to stereoisomers. Note that, except as discussed below for tautomeric forms, specifically excluded from the term "isomers," as used herein, are structural (or constitutional) isomers (i.e., isomers which differ in the connections between atoms rather than merely by the position of atoms in space). For example, a reference to a substituted 2-oxo-1-pyrrolidinyl triazole of formula (I) refers to a compound having the same covalent linkages between atoms as depicted in Formula (I)

The above exclusion does not pertain to tautomeric forms, for example, keto, enol, and enolate forms, as in, for example, the following tautomeric pairs: keto/enol, imine/enamine, amide/imino alcohol, amidine/amidine and nitroso/oxime.

Note that specifically included in the term "isomer" are compounds with one or more isotopic substitutions. For example, H may be in any isotopic form, including ¹H, ²H (D), and ³H (T); C may be in any isotopic form, including ¹²C, ¹³C, and ¹⁴C; O may be in any isotopic form, including ¹⁶O and ¹⁸O; F may be in any isotopic form, including ¹⁸F or ¹⁹F; and the like, unless otherwise specified.

The compound of formula (I) has an asymmetric centre, therefore the term "isomers" embraces all enantiomers of the compound of formula (I). The invention is to be understood to extend to the use of all such enantiomers, and to mixtures thereof in any proportion, including racemates. Formula (I) is intended to represent all individual stereoisomers and all possible mixtures thereof, unless stated or shown otherwise. In addition, compounds of formula (I) may exist as tautomers, for example keto (CH₂C=O)↔enol (CH=CHOH) tautomers or amide (NHC=O)↔hydroxyimine (N=COH) tautomers. Formula (I) is intended to represent all individual tautomers and all possible mixtures thereof, unless stated or shown otherwise.

For use in medicine, the salts of the compounds of formula (I) will be pharmaceutically acceptable salts. Other salts may, however, be useful in the preparation of the compound of formula (I) or of its pharmaceutically acceptable salts. Preferably, the compound is in its free base form. The term "free base form" refers to the uncharged molecule i.e. the molecule without any negative or positive charges (for instance protons co-ordinated to nitrogen).

Preferably, the term isomer refers to stereoisomers. Typically, the compound is a single stereoisomer or a mixture of stereoisomers. The compound may be administered as a pharmaceutically acceptable salt. Preferably, the compound is administered in its free base form.

The compound may be present in the form of a cocrystal. The term "cocrystal" refers to a crystalline single-phase material comprising at least two components which is not a solvate or a simple salt. In the field of pharmaceuticals, a cocrystal typically comprises an API and a coformer. Cocrystals of the compound are described in European patent application number 20205195.9. Thus the invention also provides cocrystals of the compound as described herein for use in the treatment of a cognitive disorder in a subject, in any of the dosage regimes as described herein.

Preferably, the compound comprises a compound of formula (Ia): 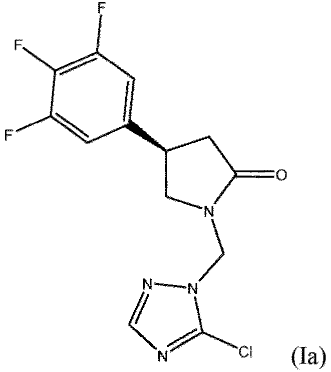 or a pharmaceutically acceptable salt thereof.

For instance, the compound may comprise at least 50% of the a compound of formula (Ia) or a pharmaceutically acceptable salt thereof, at least 60% of the a compound of formula (Ia) or a pharmaceutically acceptable salt thereof, at least 70% of the a compound of formula (Ia) or a pharmaceutically acceptable salt thereof, at least 80% of the a compound of formula (Ia) or a pharmaceutically acceptable salt thereof, at least 90%% of the a compound of formula (Ia) or a pharmaceutically acceptable salt thereof, at least 95% of the a compound of formula (Ia) or a pharmaceutically acceptable salt thereof, or at least 99% of the a compound of formula (Ia) or a pharmaceutically acceptable salt thereof.

Preferably, the compound is a substituted 2-oxo-1-pyrrolidinyl triazole of formula (I) of formula (Ia): 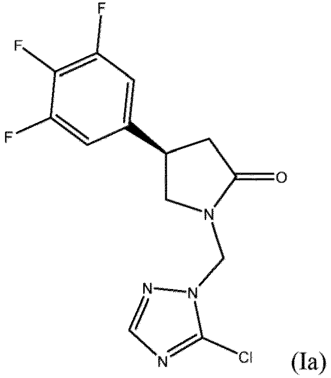 or a pharmaceutically acceptable salt thereof.

Thus, the compound may comprise (4R)-1-[(5-chloro-1H-1,2,4-triazol-1-yl)methyl]-4-(3,4,5- trifluorophenyl)pyrrolidin-2-one, or a pharmaceutically acceptable salt thereof. Preferably, the compound comprises at least 50% (4R)-1-[(5-chloro-1H-1,2,4-triazol-1-yl)methyl]-4-(3,4,5- trifluorophenyl)pyrrolidin-2-one or a pharmaceutically acceptable salt thereof, at least 60% (4R)-1-[(5-chloro-1H-1,2,4-triazol-1-yl)methyl]-4-(3,4,5-trifluorophenyl)pyrrolidin-2-one or a pharmaceutically acceptable salt thereof, at least 70% (4R)-1-[(5-chloro-1H-1,2,4-triazol-1-yl)methyl]-4-(3,4,5- trifluorophenyl)pyrrolidin-2-one or a pharmaceutically acceptable salt thereof, at least 80% (4R)-1-[(5-chloro-1H-1,2,4-triazol-1-yl)methyl]-4-(3,4,5- trifluorophenyl)pyrrolidin-2-one or a pharmaceutically acceptable salt thereof, at least 90%% (4R)-1-[(5-chloro-1H-1,2,4-triazol-1-yl)methyl]-4-(3,4,5- trifluorophenyl)pyrrolidin-2-one or a pharmaceutically acceptable salt thereof, at least 95% (4R)-1-[(5-chloro-1H-1,2,4-triazol-1-yl)methyl]-4-(3,4,5-trifluorophenyl)pyrrolidin-2-one or a pharmaceutically acceptable salt thereof, or at least 99% (4R)-1-[(5-chloro-1H-1,2,4-triazol-1-yl)methyl]-4-(3,4,5- trifluorophenyl)pyrrolidin-2-one or a pharmaceutically acceptable salt thereof.

Typically, the compound of formula (I) is (4R)-1-[(5-chloro-1H-1,2,4-triazol-1-yl)methyl]-4-(3,4,5- trifluorophenyl)pyrrolidin-2-one, or a pharmaceutically acceptable salt thereof. Preferably, the compound of formula (I) is (4R)-1-[(5-chloro-1H-1,2,4-triazol-1-yl)methyl]-4-(3,4,5- trifluorophenyl)pyrrolidin-2-one.

Provided herein is a compound which is a substituted 2-oxo-1-pyrrolidinyl triazole of formula (I) 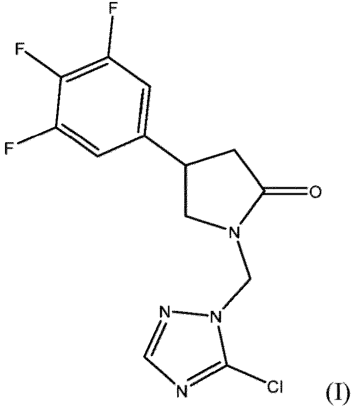 or an isomer or a pharmaceutically acceptable salt thereof, for use in the treatment of a cognitive disorder in a subject, wherein the compound is administered to the subject in an amount of from 0.2 to 5 mg.

Reference to an isomer or a pharmaceutically acceptable salt in this aspect of the invention is to be interpreted as defined above in relation to the present invention.

The compound may be administered to the subject in an amount of from 0.2 to 4 mg, from 0.2 to 3 mg, from 0.2 to 2 mg or from 0.2 to 1 mg. Preferably, the compound is administered to the subject in an amount of from 0.3 to 5 mg, from 0.3 to 4 mg, from 0.3 to 3 mg, from 0.3 to 2 mg or from 0.3 to 1 mg. The compound may be administered to the subject in an amount of from 1 to 5 mg, from 1 to 4 mg, from 1 to 3 mg or from 1 to 2 mg. Most preferably, the compound is administered to the subject in an amount of from 1 to 3 mg.

The compound may be administered to the subject in an amount of about 5 mg, an amount of about 4.5 mg, an amount of about 4 mg, an amount of about 3.5 mg, an amount of about 3 mg, an amount of about 2.5 mg, an amount of about 2 mg, an amount of about 1.5 mg, an amount of about 1 mg or an amount of about 0.5 mg. Typically, the compound is administered to the subject in an amount of about 3 mg, an amount of about 2.5 mg, an amount of about 2 mg, an amount of about 1.5 mg or an amount of about 1 mg. Preferably, the compound is administered to the subject in an amount of about 3 mg, an amount of about 2 mg or an amount of about 1 mg.

For instance, the compound may be administered to the subject in an amount of about 1 mg. The compound may be administered to the subject in an amount of about 2 mg. The compound may be administered to the subject in an amount of about 3 mg.

Preferably, the compound is administered to the subject as a single unit dosage comprising from 0.2 to 5 mg of the compound.

The term "single unit dosage" refers to a pharmaceutical formulation of the compound apportioned into a dose comprising the amount of the compound specified. A single unit dosage may therefore comprise the compound and one or more pharmaceutically acceptable excipients, as described herein.

Thus, the compound may be administered to the subject as a single unit dosage comprising from 0.2 to 4 mg, from 0.2 to 3 mg, from 0.2 to 2 mg or from 0.2 to 1 mg of the compound. Thus, preferably the compound is administered to the subject as a single unit dosage comprising from 0.3 to 5 mg, from 0.3 to 4 mg, from 0.3 to 3 mg, from 0.3 to 2 mg or from 0.3 to 1 mg of the compound. The compound may be administered to the subject as a single unit dosage comprising from 1 to 5 mg of the compound. For instance, the compound may be administered to the subject as a single unit dosage comprising an amount of from 1 to 4 mg, preferably from 1 to 3 mg, or from 1 to 2 mg of the compound.

The compound may be administered to the subject as a single unit dosage comprising an amount of about 5 mg, an amount of about 4.5 mg, an amount of about 4 mg, an amount of about 3.5 mg, an amount of about 3 mg, an amount of about 2.5 mg, an amount of about 2 mg, an amount of about 1.5 mg, an amount of about 1 mg or an amount of about 0.5 mg of the compound. Typically, the compound is administered to the subject as a single unit dosage comprising an amount of about 3 mg, an amount of about 2.5 mg, an amount of about 2 mg, an amount of about 1.5 mg or an amount of about 1 mg of the compound. More typically, the compound is administered to the subject as a single unit dosage comprising an amount of about 3 mg, an amount of about 2 mg or an amount of about 1 mg of the compound.

Preferably, the compound is administered to the subject as a single unit dosage comprising an amount of from 1 to 3 mg of the compound. For instance, the compound may be administered to the subject as a single unit dosage comprising an amount of about 1 mg of the compound. The compound may be administered to the subject as a single unit dosage comprising an amount of about 2 mg of the compound. The compound may be administered to the subject as a single unit dosage comprising an amount of about 3 mg of the compound.

The compound may be administered to the subject as multiple doses per day. For instance, the compound may be administered to the subject to provide an amount of from 0.2 to 5 mg of the compound in two or more doses. The compound may be administered to the subject to provide an amount of from 0.3 to 4 mg of the compound in two or more doses. The compound may be administered to the subject to provide an amount of from 1 to 3 mg of the compound in two or more doses. For example, a dosage of 5 mg of the compound may be administered to the subject in the form of two 2.5 mg doses.

Typically, the amount of the compound as described herein is the total amount which is administered to the subject daily, i.e. the daily dose. Preferably the amount of the compound as described herein is administered to the subject once daily. Typically, the amount of the compound, as described herein, is administered to the compound once daily as a single dose. Alternatively, the amount of the compound as described herein may be administered to the subject as multiple doses per day. For instance, a daily dose of 10 mg of the compound may be administered as two 5 mg doses, or as a single dose of 10 mg.

The compound as described herein may be administered to the subject to deliver any amount, in mg, daily, typically once per day. The compound as described herein may be administered to the subject to deliver an amount of from 0.2 to 5 mg per day of the compound, or of from 0.3 to 4 mg per day of the compound, or preferably of from 1 to 3 mg per day of the compound daily, typically once per day. The compound as described herein may be administered to the subject daily, for instance once per day, as a single unit dosage form comprising any amount of the compound as described herein. Preferably, the compound as described herein is administered once per day as a single unit dosage form comprising amount of 0.2 to 5 mg of the compound, for instance once per day as a single unit dosage form comprising an amount of 0.3 to 4 mg or more preferably once per day as a single unit dosage form comprising an amount of 1 to 3 mg of the compound.

Also provided is a compound which is a substituted 2-oxo-1-pyrrolidinyl triazole of formula (I) 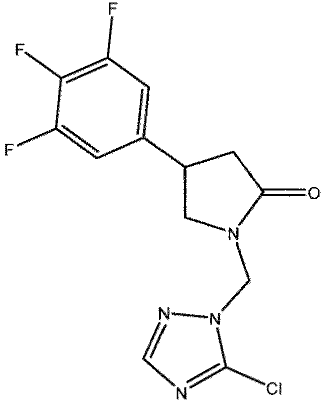 or an isomer or a pharmaceutically acceptable salt thereof, for use in the treatment of a cognitive disorder in a subject, wherein the compound is administered to the subject in an amount that provides a synaptic vesicle glycoprotein 2A (SV2A) occupancy of from 10% to 80% at trough level after once daily dosing for at least 10 days.

Typically, the SV2A occupancy is measured when the concentration of the compound in the plasma has reached a steady state. Steady state is the point at which the overall intake of a drug is in dynamic equilibrium with its elimination from the body. Typically, steady state is reached within five times the half-life of the drug after regular dosing is started. In the case of the compound of the invention, steady state is achieved within approximately 8 to 10 days.

The SV2A occupancy at trough level corresponds to the SV2A occupancy when the concentration of the compound in the plasma of the subject is at its lowest, immediately before the next dose of the compound is administered. Thus, the SV2A occupancy at trough level after once daily dosing for at least 10 days may be measured immediately before (e.g. up to one hour before) administration on the eleventh day of dosing. Put another way, the SV2A occupancy at trough level after once daily dosing for at least 10 days may be measured at least 23 hours after administration of the dose on the 10^{th} day of administration, but prior to administration of the next dose.

Reference to an isomer or a pharmaceutically acceptable salt in this aspect of the invention is to be interpreted as defined above in relation to the present invention. Preferably the compound is in its free base form.

Typically, the compound may be administered to the subject in an amount that provides an SV2A occupancy of from 10% up to (but not including) 80% at trough level after once daily dosing for at least 10 days, i.e. an occupancy of less than 80% but at least 10%. For instance, the compound may be administered to the subject in an amount that provides an SV2A occupancy of from 10% to 70% at trough level after once daily dosing for at least 10 days, or in an amount that provides an SV2A occupancy of from 10 to 79% at trough level after once daily dosing for at least 10 days, or in an amount that provides an SV2A occupancy of from 10 to 75% at trough level after once daily dosing for at least 10 days, or in an amount that provides an SV2A occupancy of from 10 to 60% at trough level after once daily dosing for at least 10 days, or in an amount that provides an SV2A occupancy of from 10 to 50% at trough level after once daily dosing for at least 10 days.

The compound may be administered to the subject in an amount that provides an SV2A occupancy of from 20 to 80% at trough level after once daily dosing for at least 10 days, an amount that provides an SV2A occupancy of from 20 to 70% at trough level after once daily dosing for at least 10 days, an amount that provides an SV2A occupancy of from 20 to 60% at trough level after once daily dosing for at least 10 days, or in an amount that provides an SV2A occupancy of from 20 to 50% at trough level after once daily dosing for at least 10 days.

SV2A occupancy may be established by using a positron emission tomography (PET) radioactive tracer such as 11C-UCB-J ((4*R*)-1-{[3-(¹¹C)Methylpyridin-4-yl]methyl}-4-(3,4,5-trifluorophenyl)pyrrolidin-2-one). SV2A occupancy may be estimated by differences in regional distribution volume between baseline and post dose PET scanning (using a Lassen plot and assuming non-displaceable distribution volume being constant). This approach gives a global estimate of brain occupancy. Once SV2A occupancies at a various blood plasma concentrations have been measured and plotted, the blood plasma level may be used to predict the SV2A occupancy. The blood plasma concentration may be established by collecting venous blood samples from the subject in lithium heparin tubes, isolating the plasma and determining the concentration of the compound using liquid chromatography coupled to mass spectrometric detection.

The compound may be administered to the subject in an amount as described herein, to achieve an SV2A occupancy as described herein. For instance, the compound may be administered to the subject daily in an amount of 0.2 to 5 mg to provide an SV2A occupancy of 10% to 80% at trough level after once daily dosing for at least 10 days. For instance, the compound may be administered to the subject daily in an amount of 1 to 3 mg to provide an SV2A occupancy of 10% to 80% at trough level after once daily dosing for at least 10 days.

The invention provides a compound which is a substituted 2-oxo-1-pyrrolidinyl triazole of formula (I) 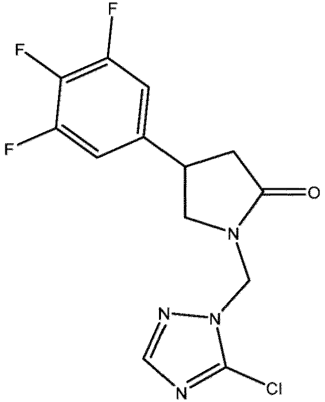 or an isomer or a pharmaceutically acceptable salt thereof, for use in the treatment of a cognitive disorder in a subject, wherein the compound is administered to the subject in an amount that provides a blood plasma concentration of the compound of from 2 to 100 ng/mL at trough level after once daily dosing for at least 10 days. For instance, the compound may be administered to the subject in an amount that provides a blood plasma concentration of the compound of from 5 to 100 ng/mL at trough level after once daily dosing for at least 10 days.

Reference to an isomer or a pharmaceutically acceptable salt in this aspect of the invention is to be interpreted as defined above in relation to the present invention.

The compound may be administered to the subject in an amount as described herein, to achieve a blood plasma concentration of the compound as described herein. For instance, the compound may be administered to the subject daily in an amount of 0.2 to 5 mg to provide a blood plasma concentration of the compound of 2 to 100 ng/mL at trough level after once daily dosing for at least 10 days.

The compound as described herein may be administered to the subject once per day to deliver an amount of the compound that provides an synaptic vesicle glycoprotein 2A (SV2A) occupancy as described herein. For instance, the compound as described herein may be administered to the subject once per day to deliver an amount of the compound that provides an synaptic vesicle glycoprotein 2A (SV2A) occupancy of from 10% to 80% at trough level after once daily dosing for at least 10 days.

The compound as described herein may be administered to the subject once per day to deliver an amount of the compound that provides a blood plasma concentration as described herein. The compound or pharmaceutical composition as described herein may be administered to the subject once per day to deliver an amount of the compound that provides a blood plasma concentration of the compound of 2 to 100 ng/mL at trough level after once daily dosing for at least 10 days.

Also provided is a pharmaceutical composition comprising a compound which is a substituted 2-oxo-1-pyrrolidinyl triazole of formula (I) 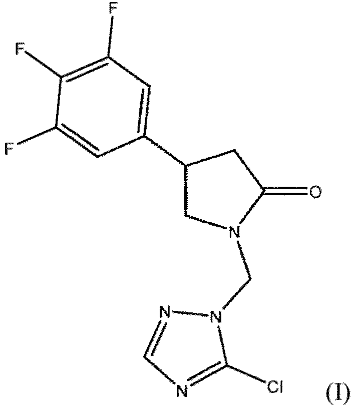 or an isomer or a pharmaceutically acceptable salt thereof, and one or more pharmaceutically acceptable excipients, for use in the treatment of a cognitive disorder in a subject, wherein the pharmaceutical composition is administered to deliver to the subject an amount of the compound as described herein.

Reference to an isomer or a pharmaceutically acceptable salt in this aspect of the invention is to be interpreted as defined above in relation to the present invention.

The pharmaceutical composition may be administered to deliver to the subject any amount of the compound, in mg, as described herein. Preferably, the pharmaceutical composition may be administered to deliver to the subject an amount of the compound of from 0.2 to 5 mg. The pharmaceutical composition may be administered to deliver to the subject an amount of from 0.2 to 4 mg, from 0.2 to 3 mg, from 0.2 to 2 mg or from 0.2 to 1 mg of the compound. The pharmaceutical composition is preferably administered to deliver to the subject an amount of from 0.3 to 5 mg, from 0.3 to 4 mg, from 0.3 to 3 mg, from 0.3 to 2 mg or from 0.3 to 1 mg of the compound. The pharmaceutical composition may be administered to deliver to the subject an amount of from 1 to 5 mg, from 1 to 4 mg, from 1 to 3 mg, or from 1 to 2 mg of the compound. Preferably, the pharmaceutical composition is administered to deliver to the subject an amount of from 1 to 3 mg. For instance, the pharmaceutical composition may be administered to deliver to the subject an amount of about 1 mg. The compound may be administered to deliver to the subject an amount of about 2 mg. The compound may be administered to deliver to the subject an amount of about 3 mg.

Typically, the pharmaceutical composition is administered to the subject as a single unit dosage comprising any amount of the compound, in mg, as described herein. For instance, the pharmaceutical composition is administered to the subject as a single unit dosage comprising from 0.2 to 5 mg of the compound. Thus, the pharmaceutical composition may be administered to the subject as a single unit dosage comprising from 0.2 to 4 mg, from 0.2 to 3 mg, or from 0.2 to 2 mg or from 0.2 to 1 mg of the compound. The pharmaceutical composition is preferably administered to the subject as a single unit dosage comprising from 0.3 to 5 mg, from 0.3 to 4 mg, from 0.3 to 3 mg, from 0.3 to 2 mg or from 0.3 to 1 mg of the compound. The pharmaceutical composition may be administered to the subject as a single unit dosage comprising from 1 to 5 mg of the compound. Typically, the pharmaceutical composition is administered to the subject as a single unit dosage comprising an amount of from 1 to 3 mg of the compound. For instance, the pharmaceutical composition may be administered to the subject as a single unit dosage comprising an amount of about 1 mg of the compound. The pharmaceutical composition may be administered to the subject as a single unit dosage comprising an amount of about 2 mg of the compound. The pharmaceutical composition may be administered to the subject as a single unit dosage comprising an amount of about 3 mg of the compound.

The pharmaceutical composition may be administered to deliver to the subject an amount of the compound that provides any synaptic vesicle glycoprotein 2A (SV2A) occupancy as described herein. For instance, the pharmaceutical composition may be administered to deliver to the subject an amount of the compound that provides any synaptic vesicle glycoprotein 2A (SV2A) occupancy of from 10% to 80% at trough level after once daily dosing for at least 10 days. The pharmaceutical composition may be administered to deliver to the subject an amount of the compound that provides any synaptic vesicle glycoprotein 2A (SV2A) occupancy of from 10% to 70% at trough level after once daily dosing for at least 10 days. The pharmaceutical composition may be administered to deliver to the subject an amount of the compound that provides any synaptic vesicle glycoprotein 2A (SV2A) occupancy of from 20% to 50% at trough level after once daily dosing for at least 10 days.

The pharmaceutical composition may be administered to deliver to the subject an amount of the compound that provides any blood plasma concentration of the compound as described herein. For instance, the pharmaceutical composition may be administered to deliver to the subject an amount of the compound that provides a blood plasma concentration of the compound of 2 to 100 ng/mL at trough level after once daily dosing for at least 10 days.

Also provided is a pharmaceutical composition comprising from 0.2 to 5 mg of the compound in the form of a single unit dose. For instance, the pharmaceutical composition may comprise 0.2 to 4 mg, from 0.2 to 3 mg, or from 0.2 to 2 mg of the compound in the form of a single unit dose. The pharmaceutical composition may comprise from 1 to 5 mg of the compound in the form of a single unit dose. Typically, the pharmaceutical composition comprises from 1 to 3 mg of the compound in the form of a single unit dose. For instance, the pharmaceutical composition may be comprise an amount of about 1 mg of the compound in the form of a single unit dose. The pharmaceutical composition may comprise an amount of about 2 mg of the compound in the form of a single unit dose. The pharmaceutical composition may comprise an amount of about 3 mg of the compound in the form of a single unit dose.

Suitable pharmaceutically acceptable excipients are well known to those skilled in the art and include pharmaceutically acceptable carriers (e.g. a saline solution, an isotonic solution), diluents, adjuvants, fillers, buffers, preservatives, anti-oxidants, lubricants, stabilisers, solubilisers, surfactants (e.g. wetting agents), masking agents, colouring agents, flavouring agents and sweetening agents. Suitable carriers, diluents, excipients, etc. can be found in standard pharmaceutical texts. See, for example, Handbook for Pharmaceutical Additives, 2nd Edition (eds. M. Ash and I. Ash), 2001 (Synapse Information Resources, Inc., Endicott, New York, USA), Remington's Pharmaceutical Sciences, 20th edition, pub. Lippincott, Williams & Wilkins, 2000; and Handbook of Pharmaceutical Excipients, 2nd edition, 1994.

The pharmaceutical composition may be suitable for oral, buccal, parenteral, nasal, topical, ophthalmic or rectal administration, or a form suitable for administration by inhalation or insufflation. Typically, the pharmaceutical composition is suitable for oral administration.

For oral administration, the pharmaceutical composition may take the form of, for example, tablets, lozenges or capsules prepared by conventional means with pharmaceutically acceptable excipients such as binding agents (e.g. pregelatinised maize starch, polyvinylpyrrolidone or hydroxypropyl methyl cellulose); fillers (e.g. lactose, microcrystalline cellulose or calcium hydrogenphosphate); lubricants (e.g. magnesium stearate, talc or silica); disintegrants (e.g. potato starch or sodium glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents, emulsifying agents, non-aqueous vehicles or preservatives. The preparations may also contain buffer salts, flavouring agents, colouring agents or sweetening agents, as appropriate.

Pharmaceutical compositions for oral administration may be suitably formulated to give controlled release of the active compound.

For buccal administration, the pharmaceutical compositions may take the form of tablets or lozenges formulated in conventional manner.

The pharmaceutical composition may be for parenteral administration by injection, e.g. by bolus injection or infusion. Formulations for injection may be presented in unit dosage form, e.g. in glass ampoules or multi-dose containers, e.g. glass vials. The compositions for injection may take such forms as suspensions, solutions or emulsions in oily or aqueous vehicles, and may contain formulatory agents such as suspending, stabilising, preserving and/or dispersing agents. Alternatively, the active ingredients may be in powder form for constitution with a suitable vehicle, e.g. sterile pyrogen-free water, before use.

In addition to the formulations described above, the pharmaceutical composition may also be formulated as a depot preparation. Such long-acting formulations may be administered by implantation or by intramuscular injection.

For nasal administration or administration by inhalation, the pharmaceutical composition may be conveniently delivered in the form of an aerosol spray presentation for pressurised packs or a nebuliser, with the use of a suitable propellant, e.g. dichlorodifluoromethane, fluorotrichloromethane, dichlorotetrafluoroethane, carbon dioxide or other suitable gas or mixture of gases.

The pharmaceutical compositions may, if desired, be presented in a pack or dispenser device which may contain one or more unit dosage forms containing the active ingredient. The pack or dispensing device may be accompanied by instructions for administration.

For topical administration the pharmaceutical composition may be conveniently formulated in a suitable ointment containing the active component suspended or dissolved in one or more pharmaceutically acceptable carriers. Particular carriers include, for example, mineral oil, liquid petroleum, propylene glycol, polyoxyethylene, polyoxypropylene, emulsifying wax and water. Alternatively, the pharmaceutical composition may be formulated as a suitable lotion containing the compound suspended or dissolved in one or more pharmaceutically acceptable carriers. Particular carriers include, for example, mineral oil, sorbitan monostearate, polysorbate 60, cetyl esters wax, cetearyl alcohol, benzyl alcohol, 2-octyldodecanol and water.

For ophthalmic administration the pharmaceutical composition may be formulated as micronized suspensions in isotonic, pH-adjusted sterile saline, either with or without a preservative such as a bactericidal or fungicidal agent, for example phenylmercuric nitrate, benzylalkonium chloride or chlorhexidine acetate. Alternatively, for ophthalmic administration the pharmaceutical composition may be formulated as an ointment such as petrolatum.

For rectal administration the pharmaceutical composition may be conveniently formulated as suppositories. These can be prepared by mixing the compound with a suitable nonirritating excipient which is solid at room temperature but liquid at rectal temperature and so will melt in the rectum to release the active component. Such materials include, for example, cocoa butter, beeswax and polyethylene glycols.

The compounds and pharmaceutical compositions described herein are typically for use in enhancing or improving cognitive ability or counteracting cognitive decline.

The terms "enhancing or improving cognitive ability" and "counteracting cognitive decline" used throughout this specification shall mean promoting cognitive function (affecting impaired cognitive function in the subject so that it more closely resembles the function of an aged-matched normal, unimpaired subject, including affecting states in which cognitive function is reduced compared to a normal subject) and preserving cognitive function (affecting normal or impaired cognitive function such that it does not decline or does not fall below that observed in the subject upon first presentation or diagnosis, e.g. to the extent of expected decline in the absence of treatment). The suitability of the compounds according to the present invention for conditions associated with enhancement or improvement of cognitive ability may be tested through assays that are well known in the art. Such assays include in particular the novel object recognition tests, the Y-maze test (for animal studies), and word recall tests and digit symbol substitution tests (DSST), such as those provided by Cogstate (for human studies).

For instance, the compound or pharmaceutical composition described herein may be for use in treating a cognitive disorder selected from autism, dyslexia, attention deficit hyperactivity disorder, obsessive compulsive disorders, psychosis, bipolar disorders, depression (major depressive disorder), Tourette's syndrome and disorders of learning in children, adolescents and adults, Age Associated Memory Impairment, Age Associated Cognitive Decline, Parkinson's Disease, Down's Syndrome, traumatic brain injury, Huntington's Disease, Progressive Supranuclear Palsy (PSP), HIV infection, stroke, vascular diseases, Pick's or Creutzfeldt-Jacob diseases, multiple sclerosis (MS), other white matter disorders and drug-induced cognitive worsening, Alzheimer's disease, schizophrenia, Lewy-bodies disease, front-temporal lobe degeneration, vascular narrowing or blockage in the brain (i.e. vascular dementia also known as multi-infarct dementia), head trauma, subjective cognitive decline and mild cognitive impairment.

The compound or pharmaceutical composition as described herein are typically for use in treating a cognitive disorder selected from subjective cognitive decline, Age Associated Memory Impairment, mild cognitive impairment, Alzheimer's disease, cognitive impairment in major depressive disorder and cognitive impairment in a subject with remitted depression following multiple episodes of major depressive disorder.

The compound or pharmaceutical composition as described herein may be for use in treating subjective cognitive decline. The compound or pharmaceutical composition as described herein may be for use in treating Age Associated Memory Impairment. The compound or pharmaceutical composition as described herein may be for use in treating mild cognitive impairment. The compound or pharmaceutical composition as described herein may be for use in treating Alzheimer's disease. The compound or pharmaceutical composition as described herein may be for use in treating cognitive impairment in major depressive disorder. The compound or pharmaceutical composition as described herein may be for use in treating cognitive impairment in a subject with remitted depression following multiple episodes of major depressive disorder.

The compound or pharmaceutical composition as described herein may be for use in treating disorders associated with loss of cognition, for example the compound or pharmaceutical composition as described herein may be for use in treating disorders in learning and memory. The compound or pharmaceutical composition as described herein may be for use in treating disorders in learning and memory, Parkinson's disease, Huntington's disease, Tourette's syndrome, and obsessive-compulsive disorder.

Typically, the compound or pharmaceutical composition as described herein is administered orally.

Typically the subject is a mammal. Preferably the subject is a human.

### EXAMPLES

### Example 1

The compound

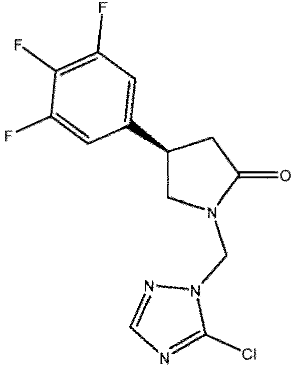

This compound may be prepared as described in WO 2015/014785.

### Example 2 - A Neuroimaging Study Employing Magnetic Resonance Imaging to Assess Brain Activity in Animals Administered a Novel Test Article in Conscious Rats

### List of abbreviations

- CNS: central nervous system
- BOLD: blood oxygen level dependent
- sGC: soluble guanylate cyclase
- CBF: cerebral blood flow
- MRI: magnetic resonance imaging
- RARE: rapid acquisition relaxation enhancement
- FOV: field of view
- HASTE: half-Fourier acquisition single-shot turbo spin echo
- TR: time to repetition
- TE: time to echo
- NEX: number of excitations

### Introduction

Synaptic vesicle protein 2A (SV2A) is a protein found in synaptic vesicles of neuronal and endocrine cells. It is part of a family of proteins, which also includes SV2B and SV2C. All members are composed of a highly N-glycosylated 80 kDa backbone organized in 12 transmembrane (TM) regions. They also present two large loops - one cytoplasmic and one luminal - and N- and C-terminal cytoplasmic sequences. SV2A is the only member of the SV2 family that is ubiquitously expressed in the adult brain in both excitatory (glutamatergic) and inhibitory (GABAergic) neurones. SV2A interacts directly with another synaptic vesicle protein, synaptotagmin-1, which acts as the calcium sensor that triggers presynaptic vesicle fusion and, thus, calcium-evoked neurotransmitter release. Studies to date suggest that SV2A knock-out (KO) reduces calcium-dependent exocytosis of neurotransmitters. SV2A KO mice that survive birth seem to be normal during their first days of life. However, after 1-2 weeks, they experience seizures, quickly followed by weight loss and death around P20. This increase in seizures appears to be due to an imbalance between excitatory and inhibitory transmission due primarily to a decrease in both the frequency and amplitude of calcium-dependent, spontaneous inhibitory post-synaptic potentials (sIPSPs), while calcium-independent, mini-IPSPs appear to be unchanged.

SV2A is the target of action of the marketed anti-epileptic drug, Keppra (levetiracetam). Its mechanism of action appears to be through a frequency-dependent inhibitory effect on neurotransmission, such that its inhibitory effect is most pronounced during bursts of high frequency firing. This probably underlies both the efficacy and tolerability of levetiracetam as an anti-convulsant.

The compound of example 1 binds with high affinity to the same site on SV2A as levetiracetam and competitively displaces the binding of levetiracetam to SV2A in vitro. However, unlike levetiracetam, and many if its analogues, the compound of example 1 is without anti-convulsant activity and competitively inhibits the anti-convulsant effect of levetiracetam in vivo. Thus, the compound of example 1 modulates SV2A function in a manner distinct to that of levetiracetam. This led to in depth studies of the in vivo pharmacological profile of the compound of example 1 in a large number of behavioural paradigms. These revealed that the compound of example 1 possesses pro-cognitive effects in a range of rodent preclinical cognitive deficit models and that these effects correlated well with the in vivo occupancy of SV2A by the compound of example 1.

In order to understand more mechanistically how the compound of example 1 modulates neuronal network activity to produce these pro-cognitive effects, a pharmaco-MRI study was performed in conscious rats to investigate how neuronal activity is modulated, in a dose-dependent way, by the compound of example 1.

### Materials and methods

Test Substance, Reference Substance(s), and Vehicle: the compound of example 1 test substance for dose-dependent change in BOLD signal was provided by Syndesi along with the formula for the vehicle.

Subjects: Forty-eight male Sprague Dawley rats weighing ca 275-300 g were obtained from Charles River Laboratories (Wilmington, MA, USA). Rat were maintained on a 12:12 hour light: dark cycle with a lights on at 0700 hours and allowed access to food and water ad libitum. All rats were acquired and cared for in accordance with the guidelines published in the Guide for the Care and Use of Laboratory Animals (National Institutes of Health Publications No. 85-23, Revised 1985) and adhered to the National Institutes of Health and the American Association for Laboratory Animal Science guidelines.

Acclimation: To reduce the stress associated with head restraint, rats were acclimated to the restraining system (head holder and body tube) one week prior to their actual imaging session. The design of the restraining system (Animal Imaging Research, Holden MA, USA) included a padded head support obviating the need for ear bars helping to reduce animal discomfort while minimizing motion artifact. These acclimation sessions were run each day for 4-5 consecutive days. Rats were briefly anesthetized with 2-3% isoflurane while being secured into the head holder. The forepaws were secured with tape. When fully conscious, the imaging system is placed into a black opaque box "mock scanner" for 30 min with a tape-recording of the MRI pulse sequence to simulate the bore of the magnet and the imaging protocol. A significant decline in respiration, heart rate, motor movements, and plasma corticosterone has been measured when the first and last acclimation periods are compared (King et al., 2005). The reduction in autonomic and somatic measures of arousal and stress improve the signal resolution and quality of the images.

Image Acquisition: Animals were scanned at 300 MHz using a quadrature transmit/ receive volume coil built into the rat head holder and restraining system for awake animal imaging (Animal Imaging Research, Holden, MA, USA). The design of the coil provided complete coverage of the brain from olfactory bulbs to brain stem with excellent B1 field homogeneity. Experiments were conducted using a Bruker Biospec 7.0 T/20-cm USR horizontal magnet (Bruker, Billerica, MA, USA) and a 20-G/cm magnetic field gradient insert (ID = 12 cm) capable of a 120-µs rise time. At the beginning of each imaging session, a high-resolution anatomical data set was collected using the RARE pulse sequence [22 slice; 1.0 mm; field of view (FOV) 3.0 cm; matrix size 256 × 256; repetition time (TR) 2.5 s; echo time (TE) 12 ms; NEX 2; 3 min acquisition time]. Functional images were acquired using a multi-slice half-Fourier acquisition single-shot turbo spin echo (HASTE) pulse sequence. Bruker Paravision automatically finds the basic frequency, shims, power requirements for 90° and 180° pulses and sets the receiver gain. A single scanning session acquired 22 slices, 1.0 mm thick, every 6.0 s (TR), using an effective TE of 48 ms, FOV 3.0 cm, matrix size 96 × 96, NEX 1, and repeated 450 times for a total scanning time of 45 min. The in-plane pixel resolution was 312 µm2.

Data Processing: Data are coregistered to a mean functional image using SPM8's coregistrational code with the following parameters: Quality: 0.97, Smoothing: 0.35mm, Separation: 0.5mm. Gaussian smoothing was performed with a FWHM of 0.8mm. Images were aligned and registered to the 3D rat brain atlas, segmented and labeled with 171 discrete anatomical regions. The alignment process was facilitated by an interactive graphic user interface. The registration process involved translation, rotation and scaling independently and in all three dimensions. Matrices that transformed each subject's anatomy were used to embed each slice within the atlas. All pixel locations of anatomy that were transformed were tagged with major and minor regions in the atlas. This combination created a fully segmented representation of each subject within the atlas. The composite statistics were built using the inverse transformation matrices. Each composite pixel location (i.e., row, column, and slice), pre-multiplied by [Ti]-1, mapped it within a voxel of subject (i). A tri-linear interpolation of the subject's voxel values (percentage change) determined the statistical contribution of subject (i) to the composite (row, column, and slice) location. The use of [Ti]-1 ensured that the full volume set of the composite was populated with subject contributions. The average value from all subjects within the group determined the composite value.

Data Analysis: Using voxel-based analysis, the percent change in BOLD signal for each independent voxel was averaged for all subjects. Each scanning session consisted of 450 data acquisitions (whole brain scans) with a period of 6 sec each for a total lapse time of 45 min. The control window was the first 50 scan repetitions (5 min baseline) while the treatment stimulation window was 50 -450 (minutes 5-45). Statistical t tests were performed on each voxel (ca. 15,000 in number) of each subject within their original coordinate system with a baseline threshold of 2% BOLD change to account for normal fluctuation of BOLD signal in the awake rodent brain (Brevard et al., 2003). As a result of the multiple t test analyses performed, a false-positive detection controlling mechanism was introduced (Genovese et al., 2002). The final results compared the last ten min of imaging (acquisitions 350-450) to the five min baseline (acquisitions 1-50). Volume of activation was compared across experimental groups using the non-parametric Wilcoxon signed rank test statistic. The brain areas were ranked in order of their significance. Brain areas were considered statistically different between experimental groups when comparison produced P-values less than or equal to our cutoff of 0.05. Post hoc analyses were performed with a non-parametric Kruskal-Wallis Test.

**Study design:**

| **Grou p no.** | **N** | **Treatment** | **Dose (mg/kg )** | **Concentratio n** | **Volum e** | **RO A** | **Imaging** |
|---|---|---|---|---|---|---|---|
| 1 | 12 | Methyl Cellulose 1% (in H2O), Tween 80 0.1%, Antifoam 0.1% | DVE | NA | 5 mL/kg | I.P. | Images will be acquired continuousl y starting 5 min prior to dosing and for 40 minutes post-dosing |
| 2 | 12 | compound of example 1 | 0.1 | 0.02 mg/mL | | | |
| 3 | 12 | compound of example 1 | 0.3 | 0.06 mg/mL | | | |
| 4 | 12 | compound of example 1 | 1.0 | 0.2 mg/mL | | | |

| | | | | | | | |
|---|---|---|---|---|---|---|---|
| No. = number; ROA = route of administration | | | | | | | |

On the day of imaging rats were lightly anesthetized with 2-3% isoflurane as described in Acclimation, fixed with a IP catheter, secured into the awake rodent imaging device, and positioned in the magnet. Rats were allowed to recover from anesthesia (ca 20 min) before the start of imaging. Rats were randomly assigned to one of four groups corresponding to vehicle, 0.1, 0.3, or 1.0 mg/kg of the compound of example 1. To deliver drug remotely during the imaging session, a poly-ethylene tube (PE-20), approximately 30 cm in length was connected to the I.P. catheter. At the start of image acquisition 51, drug or vehicle was infused over 1 min. Over the course of the study data from five of the 48 rats were lost to motion artifact, and technical artifact. The final numbers were Veh (n=10), 0.1 mg (n=11), 0.3mg (n=11), 1.0mg (n=11).

Assay Conditions: No blood or brain tissue was collected post imaging for drug assay.

### Results

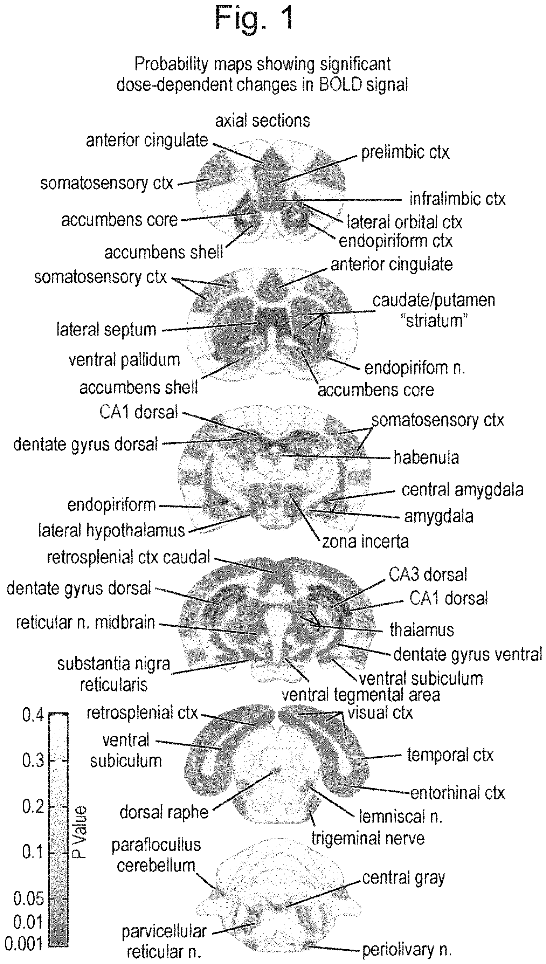

The median (Med) number of positive voxels activated for both vehicle and the compound of example 1, at 20-30 min post injection are shown in the Tables 1 and 2. These tables are truncated showing only those areas that were significantly different from 171 brain regions. These brain areas are ranked in order of their significance. Table 1 includes comparisons for all four treatments (vehicle, 0.1, 0.3, 1.0 mg/kg of the compound of example 1) while Table 2 shows only data from low dose of the compound of example 1. In Table 1, 71/171 brains areas were significantly affected by the four treatments. Areas associated with learning and memory i.e., hippocampal complex are marked with a * while areas associated reward and motivation i.e., basal ganglia/mesencephalic dopaminergic system are marked with a #. A global heat map highlighting the significant areas in 2D axial sections is shown in Fig 1. The dose response presents as an inverted U with the low dose of 0.1 mg/kg having the greatest effect on the volume of activation while the high dose, 1.0 mg/kg, presents with little or no activation. In Table 2 comparing the low dose of the compound of example 1 to vehicle there are 30/171 significantly activated. Again, there is activation in the basal ganglia (marked with a #) and hippocampal complex (marked with a *) with several areas of the thalamus (zona incerta, subthalamic, ventral medial, parafascicular, lateral posterior, posterior and pretectal thalamic nuclei) and somatosensory and motor cortices. These thalamic and cortical areas with the striatum (caudate/putamen) comprise the cortico-basal ganglia-thalamocortical loops involved motivation, motor control, memory and cognition.

**Table 1 Shown are the median number (Med) of positive voxels 20-30 min post injection of Veh, 0.1, 0.3, 1.0 mg/kg - positive BOLD volume of activation**

| **Brain Area** | **Veh Med** | **0.1 Med** | **0.3 Med** | **1 Med** | **P value** |
|---|---|---|---|---|---|
| supraoptic nucleus | 0 | 0 | 2 | 0 | 0.001 |
| premammillary nucleus | 2 | 1 | 3 | 0 | 0.001 |
| CA1 dorsal * | 0 | 23 | 15 | 0 | 0.001 |
| subiculum dorsal * | 3 | 13 | 12 | 0 | 0.001 |
| dentate gyrus dorsal * | 6 | 17 | 9 | 0 | 0.002 |
| central amygdaloid nucleus | 3 | 8 | 4 | 0 | 0.003 |
| accumbens core # | 0 | 13 | 3 | 0 | 0.003 |
| lateral orbital ctx | 12 | 29 | 14 | 1 | 0.003 |
| paraventricular nucleus | 0 | 4 | 5 | 0 | 0.003 |
| lateral septal nucleus* | 11 | 34 | 35 | 4 | 0.004 |
| endopiriform nucleus | 0 | 2 | 0 | 0 | 0.004 |
| ventral lateral striatum # | 11 | 82 | 1 | 0 | 0.005 |
| reticular nucleus midbrain | 8 | 19 | 18 | 1 | 0.006 |
| lateral preoptic area | 0 | 12 | 3 | 0 | 0.006 |
| infralimbic ctx # | 2 | 19 | 21 | 4 | 0.006 |
| habenula nucleus | 2 | 11 | 10 | 0 | 0.006 |
| substantia nigra reticularis # | 6 | 19 | 22 | 2 | 0.006 |
| visual 1 ctx | 0 | 27 | 21 | 0 | 0.007 |
| dorsal raphe | 0 | 1 | 0 | 0 | 0.007 |
| retrosplenial caudal ctx | 5 | 43 | 27 | 0 | 0.007 |
| dentate gyrus ventral * | 4 | 14 | 14 | 0 | 0.008 |
| ventral medial striatum # | 6 | 47 | 18 | 0 | 0.008 |
| ventral subiculum * | 7 | 26 | 25 | 3 | 0.008 |
| accumbens shell # | 4 | 8 | 10 | 2 | 0.009 |
| ventral tegmental area # | 0 | 8 | 5 | 0 | 0.009 |
| lateral posterior thalamic nucleus | 0 | 11 | 3 | 0 | 0.009 |
| dorsal medial striatum # | 4 | 39 | 30 | 0 | 0.01 |
| anterior cingulate area | 11 | 67 | 37 | 2 | 0.01 |
| granular cell layer | 41 | 70 | 52 | 9 | 0.011 |
| lateral hypothalamus | 12 | 56 | 59 | 2 | 0.011 |
| anterior pretectal nucleus | 1 | 7 | 5 | 0 | 0.013 |
| prelimbic ctx # | 15 | 50 | 32 | 4 | 0.013 |
| ventral orbital ctx | 2 | 19 | 4 | 1 | 0.015 |
| zona incerta | 0 | 9 | 3 | 0 | 0.015 |
| triangular septal nucleus * | 1 | 4 | 1 | 0 | 0.015 |
| lateral amygdaloid nucleus | 0 | 5 | 0 | 0 | 0.015 |
| periolivary nucleus | 0 | 3 | 12 | 0 | 0.017 |
| parafascicular thalamic nucleus | 4 | 20 | 12 | 0 | 0.018 |
| extended amydala | 0 | 8 | 2 | 0 | 0.018 |
| dorsal lateral striatum # | 8 | 80 | 12 | 0 | 0.02 |
| visual 2 ctx | 0 | 51 | 21 | 0 | 0.021 |
| bed nucleus stria terminalis | 3 | 29 | 13 | 0 | 0.022 |
| basal amygdaloid nucleus | 4 | 22 | 4 | 0 | 0.023 |
| CA3 dorsal * | 2 | 4 | 4 | 0 | 0.023 |
| root of trigeminal nerve | 6 | 22 | 29 | 6 | 0.024 |
| medial geniculate | 1 | 3 | 2 | 0 | 0.024 |
| primary somatosensory ctx upper lip | 2 | 39 | 5 | 0 | 0.025 |
| paraventricular nucleus | 2 | 4 | 3 | 0 | 0.026 |
| entorhinal ctx * | 49 | 134 | 75 | 5 | 0.027 |
| medial amygdaloid nucleus | 2 | 18 | 13 | 1 | 0.028 |
| ventromedial thalamic nucleus | 0 | 10 | 2 | 0 | 0.029 |
| primary somatosensory ctx shoulder | 1 | 2 | 0 | 0 | 0.03 |
| prerubral field | 0 | 1 | 0 | 0 | 0.031 |
| primary somatosensory ctx jaw | 27 | 64 | 20 | 6 | 0.032 |
| central gray | 0 | 3 | 3 | 0 | 0.033 |
| CA2 * | 0 | 0 | 0 | 0 | 0.034 |
| posterior hypothalamic area | 1 | 19 | 15 | 0 | 0.035 |
| primary somatosensory ctx barrel field | 1 | 29 | 7 | 0 | 0.039 |
| temporal ctx * | 0 | 5 | 1 | 0 | 0.039 |
| paraflocculus cerebellum | 21 | 66 | 42 | 8 | 0.04 |
| parvicellular reticular n. | 6 | 5 | 24 | 4 | 0.041 |
| primary somatosensory ctx forelimb | 4 | 39 | 9 | 3 | 0.041 |
| substantia innominata | 0 | 0 | 0 | 0 | 0.042 |
| lemniscal nucleus | 4 | 12 | 10 | 1 | 0.042 |
| medial orbital ctx | 7 | 15 | 12 | 2 | 0.043 |
| posterior thalamic nucleus | 0 | 11 | 0 | 0 | 0.044 |
| copula of the pyramis | 0 | 0 | 11 | 0 | 0.044 |
| auditory ctx | 3 | 27 | 8 | 0 | 0.044 |
| reuniens nucleus * | 0 | 14 | 11 | 0 | 0.045 |
| ventral pallidum # | 0 | 9 | 3 | 0 | 0.048 |
| medial cerebellar nucleus fastigial | 0 | 0 | 0 | 0 | 0.048 |

**Table 2. Shown are the median number (Med) of positive voxels 20-30 min post injection of 0.1, mg/kg of the compound of example 1versus Vehicle - positive BOLD volume of activation**

| Brain area | Veh Med | 0.1 Med | P value |
|---|---|---|---|
| CA1 dorsal * | 0 | 23 | 0.008 |
| subthalamic nucleus | 0 | 0 | 0.017 |
| zona incerta | 0 | 9 | 0.019 |
| prerubral field | 0 | 1 | 0.021 |
| ventral tegmental area # | 0 | 8 | 0.024 |
| magnocellular preoptic nucleus | 0 | 1 | 0.029 |
| granular cell layer | 41 | 70 | 0.032 |
| extended amydala | 0 | 8 | 0.032 |
| auditory ctx | 3 | 27 | 0.032 |
| visual 1 ctx | 0 | 27 | 0.033 |
| ventral medial striatum # | 6 | 47 | 0.036 |
| ventromedial thalamic nucleus | 0 | 10 | 0.036 |
| ventral lateral striatum # | 11 | 82 | 0.036 |
| endopiriform nucleus | 0 | 2 | 0.036 |
| primary somatosensory ctx trunk | 0 | 2 | 0.036 |
| substantia nigra reticularis # | 6 | 19 | 0.037 |
| parafascicular thalamic nucleus | 4 | 20 | 0.037 |
| lateral posterior thalamic nucleus | 0 | 11 | 0.037 |
| primary motor ctx | 22 | 119 | 0.038 |
| CA2 * | 0 | 0 | 0.039 |
| ventral pallidum # | 0 | 9 | 0.043 |
| primary somatosensory ctx forelimb | 4 | 39 | 0.043 |
| secondary somatosensory ctx | 1 | 16 | 0.043 |
| lateral septal nucleus * | 11 | 34 | 0.045 |
| central amygdaloid nucleus | 3 | 8 | 0.047 |
| anterior pretectal nucleus | 1 | 7 | 0.047 |
| infralimbic ctx # | 2 | 19 | 0.047 |
| primary somatosensory ctx barrel field | 1 | 29 | 0.048 |
| lateral hypothalamus | 12 | 56 | 0.048 |
| posterior thalamic nucleus | 0 | 11 | 0.05 |

### Conclusion

Rats given the compound of example 1 show a change in brain activity as determined by volume of activation and BOLD signal change over time. The activity was primarily positive BOLD with very little negative BOLD. The lowest dose (0.1 mg/kg i.p., i.e. low SV2A receptor occupancy, approximately 20-50% occupancy 60 minutes post adminstration) produced the greatest effect.

When examining the low dose effect alone there was a significant activation in brain areas associated with the cortico-basal ganglia-thalamocortical loops. Disruption in these integrated brain areas is associated with multiple clinical disorders e.g. disorders in learning and memory, Parkinson's disease, Huntington's disease, Tourette's syndrome, and obsessive-compulsive disorder. The fact that the lowest dose (0.1 mg/kg i.p.) had the greatest effect implies that it is not necessary to have complete SV2A receptor occupancy in order to achieve the strongest pro-cognitive effects. Indeed, the results indicate that for therapies intended to improve cognition and memory, lower SV2A receptor occupancies may be more useful.

### Example 3 - A First-in-Human, Randomized, Placebo-controlled, Single Ascending Oral Dose Study of the compound of example 1 in Healthy Male Subjects including Receptor Occupancy Measurements after Single Dose of the compound of example 1 and an Assessment of Food Effect

### Materials

All images were acquired using the PET/MR Scanner: GE Signa TOF PET-MR 3Tesla with software version MP26. As gamma counter, a 1480 Wizard well type gamma counter met 3-inch NaI crystal was used.

[¹¹C]-UCB-J is a PET tracer for imaging the synaptic vesicle glycoprotein 2A in the human brain. [¹¹C]-UCB-J is (4*R*)-1-{[3-(¹¹C)Methylpyridin-4-yl]methyl}-4-(3,4,5-trifluorophenyl)pyrrolidin-2-one.

### Image acquisition and procedures

Participants arrived approximately 90 minutes prior to the scheduled scan. This allowed the positioning of all necessary peripheral catheters before the start of the PET scan.

Prior to PET imaging, at the PET centre, an indwelling venous cannula was inserted into an antecubital vein of the forearm to inject the radioligand. When required, an indwelling arterial catheter was placed in the radial artery of the opposite arm (if not feasible, can be in same arm) by a trained physician after numbing the area with a local anaesthetic prior to placement to withdraw blood for the measurement of the PET input function. On each PET scan day, no more than 3 skin penetrations per arm and one arterial penetration per arm were permitted before trying in the other arm. The venous catheter was used for the injection of [¹¹C]-UCB-J. [¹¹C]UCB-J was administered as an i.v. bolus into the antecubital vein over approximately 20 seconds. The tracer volume was between 6 ml and 12 ml. With the pump system configured to inject 0.33 ml per second, tracer injection time varied between 18 sec and 36 sec. The arterial catheter was used for collection of arterial blood samples and no participant received more than two arterial catheterizations during his participation in the study. An arterial catheter was maintained for up to 2 days depending on evaluation by medical staff. In case two arterial catheters were placed during the study conduct, then placement in alternate wrists was done. If the medical staff decided to remove an arterial catheter at any given time, a new one was placed for a next scan taking into account that no more than two arterial catheterizations per participant were allowed. Participants were asked to void their bladder before the start of each PET scan. Participants were positioned in the PET scanner so that scans were acquired in 3D list-mode in one bed position and the entire brain volume was included in the field of view. At screening and simultaneous with the PET acquisition, both a 3D volumetric T1-weighted BRAVO sequence (plane: sagittal; TE: 3.2ms; TR: 8.5ms; TI: 450ms; Flip Angle: 12; Receiver Bandwidth: 31.2; NEX: 1; voxelsize: 1x1x1 mm) and 3D T2-weigthed CUBE FLAIR sequence (plane: sagittal direction; TE: 137ms; #echoes: 1; echo train length: 190; TR: 8500ms; TI: 50ms; Receiver Bandwidth: 31.25; NEX: 1; voxelsize: 1.2x1.3x1.4mm) were acquired to detect possible structural abnormalities, and serve as an anatomical reference to define ROIs when co-registered with the PET scan. To correct for attenuation, a Zero-Echo Time (ZTE) scan was taken to allow the generation of a MR based attenuation map. At intermediate time points during acquisition, the ZTE sequence was repeated to have additional data for adjusting the MR based attenuation map in case of severe head motion. Next to anatomical MR sequences, eASL and rs-fMRI data were acquired simultaneously with the PET data, to determine regional cerebral blood flow and regional interactions in the brain in a resting or task-negative state. At least one eASL and one rs-fMRI sequence were performed during the PET acquisition but these sequences were repeated if time allowed. The total duration of the emission scan was 90 minutes. PET data will be rebinned in time frames according to the following scheme: 6x15s - 3x30s - 3x60s - 2x90s - 2x3min - 9x5min - 3x10min

A summary of the PETMR datasets are listed in Table 3.

**Table 3: Summary of MRI and PET scan parameters**

| **Table 3: Summary PET scan parameters** | |
|---|---|
| Dynamic PET | Co-incidences will be detected from the time of injection until the end of the scan (90 minutes) |
| T1 BRAVO | High resolution T1 weigthed MR, used for anatomical reference |
| T2 FLAIR | High resolution T2 weigthed MR with Fluid Attenuated Inversion Recovery, used for anatomical reference |
| eASL | Enhanced Arterial Spin Labelling MR, used to determine the regional cerebral blood flow |
| RS-fMRI | Resting state fMRI, using BOLD functional MRI to to evaluate regional interactions that occur in a resting or task-negative state |
| ZTE | MR based Attenuation Correction, used to determine MR-based attenuation correction and to correct for motion during the scan. |

The emission PET scan for each participant was corrected for deadtime, randoms and scatter and reconstructed using an iterative (OSEM - ordered subsets expectation maximization, 4 iterations, 28 subsets) algorithm modeling the detector response and using time of flight information. Data were reconstructed using a matrix size of 192 by 192 and 89 slices with an in-plane pixel size of 1.56 mm and a slice separation of 2.78 mm. Two reconstructed datasets were generated, one dataset which is postsmoothed with a Gaussian filter (4.5 mm Full Width Half Maximum - FWHM) and one dataset with no postfiltering applied.

### Monitoring and blood sampling

Arterial blood samples were taken to reconstruct an arterial input function during the PET scanning procedure. This was done by manual blood sampling (2 mL discrete samples - lithium heparin-containing tubes (LiH)). Nineteen (19) arterial samples were taken at the following time points post injection (p.i.) : 10sec - 20sec - 30sec - 40sec - 50sec - 1min - 1min10sec - 1min20sec - 1min30sec - 1min40sec - 2min - 2min30sec - 3min - 5min - 15min - 30min - 45min - 60min - 80min p.i.

Additional 6 arterial blood samples (4 mL samples - LiH) for HPLC metabolite analysis were taken at: 5, 10, 15, 30, 45 and 60 min p.i.

In addition to these, arterial samples (4 mL LiH tubes) for the compound of example 1pharmacokinetics were taken and processed as per laboratory manual at predose (T0) and then right before starting PET scanning (i.e. right before [¹¹C]-UCB-J injection) , at 30 minutes, 60 minutes and 90 minutes p.i. at each PET scan procedure (in total 9 samples in case of 2 postdose PET scans or 5 in case of 1 postdose PET scan). Labelled tubes were available for the collection of the samples. After collection of the blood samples, they were transported to the Radiopharmacy lab for further processing.

**Table 4: Time and event schedule PET procedure**

| **Table 4: Timing PET related procedures** | | |
|---|---|---|
| ***Time*** | ***Start Scan 0h*** | ***End Scan 1h30min*** |
| Dose of [¹¹C]-UCB-J | X | |
| Void Bladder ^{(d)} | X | X |
| Supine Position in Bed | X | |
| PET/MR Scan | X | |
| 2mL Arterial Blood Samples [¹¹C]-UCB-J Activity ^{(a, b)} | Discrete manual blood sampling procedure: | |
| | 10sec - 20sec - 30sec - 40sec - 50sec - 1min - 1min10sec - 1min20sec - 1min30sec - 1min40sec - 2min - 2min30sec - 3min - 5min - 15min - 30min - 45min - 60min - 80min | |
| 4mL Arterial Blood Samples [¹¹C]-UCB-J Metabolites Analysis ^{(a, b)} | 5 min, 10 min, 15 min, 30 min, 45 min, 60 min^{(c)} | |
| 4mL Li Hep PK blood samples (compound of example 1) | prescan, 30 min, 60 min, 90 min | |
| Drink Glass of Water | | X |

| | | |
|---|---|---|
| a) Tracer time points and number of samples, but not total blood volume, can be changed based on observed tracer kinetics b) Times are post injection of [¹¹C]-UCB-J c) but with a maximum of 6 metabolite samples d) Participants should void their bladder before and after the PET scan. | | |

### Radiotracer: [¹¹C]-UCB-J

### Dose, injection method and radiation dosimetry

370 MBq [¹¹C]-UCB-J was administered as an i.v. bolus into the antecubital vein over at least 18 seconds, followed by saline flush. In case of insufficient production yield, activity down to 50% of the target activity was used at the principal investigator's discretion. The exact amount of injected volume, mass and activity were recorded.

### Packaging, labeling and storage conditions

The i.v.-microdose of [¹¹C]-UCB-J was diluted in a final batch volume of 12 mL, consisting of 11 mL saline (0.9%) and 1 mL ethanol. Preparation and quality control of the [¹¹C]-UCB-J i.v.-solution was performed by the PET-RadioPharmacy lab, UZ-Leuven Campus Gasthuisberg. The study drug was QP released to the Department of Nuclear Medicine with appropiate labeling.

The radiochemical purity of the tracer was ≥95%. All radiolabelled study drugs must be stored at controlled room temperatures ranging from 15°C to 25°C and must be used within 1 hour after preparation. Therefore, [¹¹C]-UCB-J was stored for maximal 1 hour at room temperature.

### Quality control

After synthesis, [¹¹C]-UCB-J was stored for maximal 1 hour at room temperature and as subjected to a quality control. Afterwards, the compound was released by the radiopharmacist, according to standard operating procedures for the release of an in-house prepared radiopharmaceutical. Unused tracer was retained in the product bottle inside the lead container to allow for decay. After that, the content and the bottle containing unused tracer were used for sterility and endotoxins testing.

### Analysis of PET results

Input function: Once Arterial blood samples were collected (in lithium heparin-containing tubes), the blood was centrifuged to separate the plasma. This resulted in 19 blood samples and 19 plasma samples around 0.5 grams. All samples were weighed and immediately counted for radioactivity in the automated gamma counter with a measurement time of a 20 seconds per sample. To obtain input function values, the gamma counter measurements were normalized for efficiency by means of a calibration factor obtained by measuring simultaneously a calibration sample of an isotope with a long half-life (68Ge with 272 days half-life) and known activity. After applying this calibration factor, the activity was divided by the weight of the corresponding sample and corrected for decay between the start of the PET scan and the start of gamma counter measurements. This way input function values were obtained in kBq/cc for various time points during the PET scan. If appropriate, a multi-exponential was fitted to the input function measurements to reduce noise effects.

Metabolite analysis: As for the input function, arterial samples were collected in lithium heparin-containing tubes. Next, the blood was centrifuged to separate the plasma. Plasma were analyzed and quantified using Reversed-Phase High Performance Liquid Chromatography (RP-HPLC) for the presence of radiometabolites. These plasma samples were spiked with 20 µL of authentic [¹¹C]-UCB-J and analyzed using RP-HPLC. After passing through an in-line UV detector and a 3 inch NaI(Tl) scintillation detector, the HPLC eluate was collected in two fractions. Fraction n° 1 represents the polar metabolite(s), fraction n°2 consists of the intact [¹¹C]-UCB-J can be stored for maximal 1 hour at room temperature and possible apolar metabolite(s). As such a total of 12 aliquots were obtained (6 for each fraction) and the radioactivity in both fractions was measured using an automated gamma counter for 20 seconds per sample. If appropriate, a multi-exponential/Hill function was fitted to the parent fraction measurements to reduce noise effects.

Regional brain uptake: Dynamic PET data were checked and corrected for motion by performing a rigid frame by frame co-registration with an average frame of the first 15 frames representing the first 15 min of the PET acquisition. Next, PET images were co-registered rigidly with MRI (translation and rotation only). A Statistical Parametric Mapping based multichannel segmentation (SPM12, standard settings) using both the 3D T1 BRAVO and interpolated T2 CUBE FLAIR determined subject specific tissue probability maps for gray matter, white matter and cerebrospinal fluid. The PMOD Neuro Tool (v.4.0, PMOD Inc, Zurich, Switzerland) was used for the automatic extraction of regional Time Activity Curves (TACs) by projecting a simplified Hammers atlas on the dynamic PET data. For this purpose, dynamic PET data are spatially aligned to Montreal Neurological Institute (MNI) space (altas space) using the 3D T1 BRAVO data. This spatial transformation was used to project the VOIs of the brain atlas, defined in MNI space, to PET space. Volume of Interests (VOIs) are restricted to gray matter by applying a simple threshold of 0.3 to the individual gray matter probability maps, thus taking advantage of the subject specific gray matter probability map resulting from an SPM-based multichannel MR segmentation. This way, volumes of interest (VOIs) were defined in PET space for the frontal, temporal, parietal and occipital cortex as well as the insula, anterior and posterior cingulate, striatum, thalamus, hippocampus , cerebellum, and brainstem. From all cortical VOIs a composite cortical VOI was created to extract overall cortical TACs from the dynamic PET data.

Kinetic modeling was performed using an arterial blood and metabolite corrected arterial plasma input function obtained as described previously. As demonstrated by the results of previous study, a one tissue reversible model (1TCM and Logan plot for parametric mapping) is the most appropriate model to describe [¹¹C]-UCB-J brain kinetics and therefore this model was used to estimate the distribution volume of [¹¹C]-UCB-J in brain tissue. These regional [¹¹C]-UCB-J distribution volumes were considered as the main quantitative endpoints. However, next to regional distribution volumes based on arterial input functions, a simplified reference tissue model (SRTM) with subcortical white matter as reference tissue was used to determine regional binding potential values. In case of problems with the arterial line, these regional [¹¹C]-UCB-J binding potentials were considered as the main quantitative endpoints instead of regional distribution volumes. The occupancy of the compound of example 1 was estimated by differences in regional distribution volume between baseline and post dose PET scanning (using a Lassen plot and assuming non-displaceable distribution volume being constant). This approach gives a global estimate of brain occupancy. Next to this global estimate, regional binding potential values were used to obtain regional occupancy estimates. In case of problems with the arterial line, regional binding potential values were also be used to estimate global brain occupancy.

PK/PD modeling: Receptor occupancy data was analysed as a function of the plasma concentration of the compound of example 1 using a suitable non-linear mixed effect model.

### Results

The table below shows the measured receptor occupancy in response to different doses of the compound of example 1 at tmax (2 hours), 22 hours and 26 hours post dose.

**Table 5: receptor occupancies in response to different doses at time points of 2 hours, 22 hours or 26 hours**

| **Dose compound of example 1** | **tmax (2h)** | **22h** | **26h** |
|---|---|---|---|
| 1mg | 25% | below level of detection | |
| 1mg | 21% | below level of detection | |
| 10mg | 69% | 50% | |
| 10mg | 68% | 46% | |
| 20mg | 76% | | 56% |
| 20mg | 76% | | 66% |
| 2 mg | 42% | 19% | |
| 2 mg | 39% | 20% | |

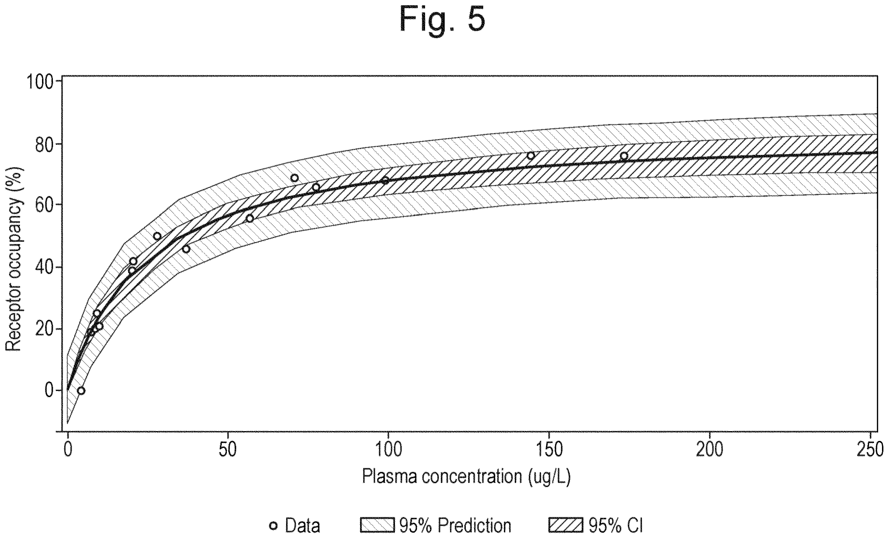

Figure 5 shows a plot of plasma concentration vs receptor occupancy for the various doses. The EC50 value was calculated as 24.9ng/mL (see Table 6).

**Table 6:**

| **Parameter estimates from PK/occupancy model** | | | | |
|---|---|---|---|---|
| **Parameter** | **Mean** | **Standard Deviation** | **95% HPD Interval** | |
| **e0 (fixed)** | 0 | | | |
| **ec50** | 24.9 | 3.9 | 17.8 | 33.3 |
| **emax** | 84.9 | 4.2 | 76.5 | 93.5 |

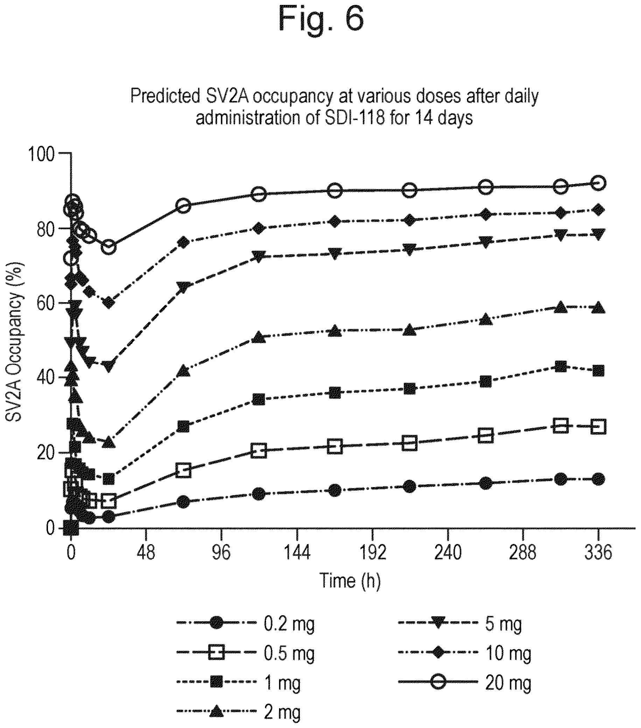

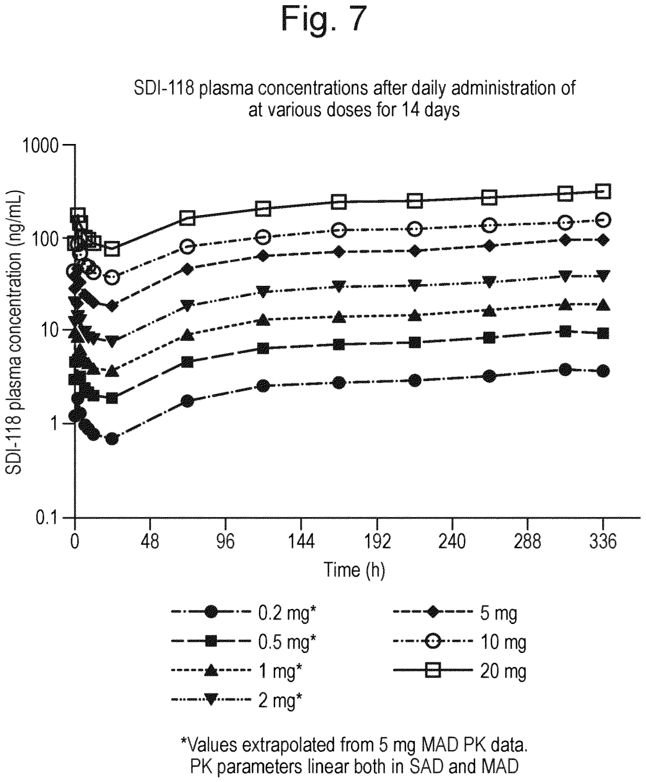

Figure 6 shows repeat dose pharmacokinetic data showing a calculation of SV2A occupancy based on measured plasma levels of the compound of example 1. The data show that for all doses, steady state was reached within approximately 8 to 10 days. SV2A occupancy for the 5, 10 and 20 mg doses were calculated from plasma level data from the dosage study described above. The data points for lower doses were extrapolated from the experimental data for the 5mg dose on the basis of the observed linear pharmacokinetics. After day 1, where there were multiple sampling timepoints, the graph shows trough SV2A occupancy at trough plasma levels. Figure 7 shows the plasma levels of the compound of example 1 used as the basis for calculating the SV2A occupancies shown in Figure 6.

Details on the adverse events occurring in the study is provided below. As may be seen the doses of interest were safe and well tolerated.

### Table 7 Summary Table of Treatment-Emergent Adverse Events in multiple dose study of SDI-118 in young male subjects

| **Treatment-Emergent Adverse Events, n (%)** | **Placebo** | **SDI-118 5 mg** | **SDI-118 10 mg** | **SDI-118 20 mg** | **All SDI-118** |
|---|---|---|---|---|---|
| | **N = 6** | **N = 6** | **N = 6** | **N = 6** | **N = 18** |
| At least one TEAE | 4 (66.7) | 0 | 2 (33.3) | 4 (66.7) | 6 (33.3) |
| At least 1 TEAE possibly related to treatment | 1 (16.7) | 0 | 0 | 4 (66.7) | 4 (22.2) |
| At least 1 TEAE probably related to treatment | 1 (16.7) | 0 | 1 (16.7) | 0 | 1 (5.6) |
| At least 1 TEAE definitively related to treatment | 0 | 0 | 0 | 0 | 0 |

| | | | | | |
|---|---|---|---|---|---|
| N = number of subjects with data; n = number of subjects with this observation, SAE = serious adverse event; TEAE = treatment-emergent adverse event The denominator for the percentage calculations is the total number of subjects per treatment and per analysis phase and period in the safety analysis set. Source: Table 14.3.1.1 | | | | | |

This study identifies dosages of the compound of example 1 in humans that provide a variety of receptor occupancies. In particular, this study indicates the doses required to provide partial (10 to 80%) SV2A occupancy. These lower doses may provide useful pro-cognitive effects, as evidenced by example 2, and may have utility in treating multiple clinical disorders associated with loss of cognition e.g. disorders in learning and memory, Parkinson's disease, Huntington's disease, Tourette's syndrome, and obsessive-compulsive disorder.

### Example 4 - resting state BOLD-fMRI

This example describes the fMRI analysis performed in the context of the study in Example 3. Simultaneous with the dynamic PET described in example 3 above, MRI data were acquired using a resting state BOLD-fMRI (rs-fMRI) protocol. In the following section, the methodology and results of the analysis of these MRI data are presented.

### Methodology - Resting state BOLD-fMRI

Resting state BOLD-fMRI data were acquired according to the following protocol:

For the analysis of the rs-fMRI scans, all data were converted to the BIDS format for processing with the CONN toolbox (release 19c).

The following preprocessing steps were performed:
- Slice timing correction: Temporal misalignment between different slices of the rs-fMRI scans, due to the sequential nature of the fMRI acquisition, is corrected by applying an appropriate timeshift to match the actual acquisition time
- Realignment: RS-fMRI data were realigned where all scans were coregistered to the first scan of the first session. This way, potential susceptibility distortion-by-motion interactions were also addressed by estimating the derivatives of the deformation field with respect to head movement, and resampling the functional data to match the deformation field of the reference image.
- Outlier detection: Potential outlier scans were identified from the observed global BOLD signal and the amount of subject-motion in the scanner. These outlier scans were taken into account in the denoising step.
- Direct simultaneous segmentation (GM-WM-CSF) and MNI (spatial) normalization: RS-fMRI data were normalized into standard MNI space and segmented into grey matter, white matter, and CSF tissue classes using an unified segmentation and normalization procedure. For this purpose, the mean BOLD signal was used as reference image. Tissue classes were used for further denoising.
- Smoothing: RS-fMRI data were smoothed with a Gaussian kernel of 8mm full width half maximum (FWHM), in order to increase BOLD signal-to-noise ratio and reduce the influence of residual variability in functional and gyral anatomy across subjects.
- Denoising: Potential confounding effects to the estimated BOLD signal were estimated and removed separately for each voxel and for each subject and functional scanning session. This was done using Ordinary Least Squares (OLS) regression to project each BOLD signal time series to the sub-space orthogonal to all potential confounding effects. Potential confounding effects included noise components from cerebral white matter and cerebrospinal areas, estimated subject-motion parameters and identified outlier scans. Temporal band-pass filtering was applied by removing temporal frequencies below 0.008 Hz or above 0.09 Hz from the BOLD signal in order to focus on slow-frequency fluctuations while minimizing the influence of physiological, headmotion and other noise sources. Temporal band-pass filtering was performed after the nuisance regression procedure in order to avoid any frequency mismatch in the regression of the confounding effects.

After these pre-processing steps, a data driven approach was taken: A group-ICA (independent component analysis) approach was applied consisting of a fastICA algorithm for group-level independent component definition and GICA3 for subject-level backprojection. This way, dimensions were reduced to a predefined number of 32 independent components which were then spatially matched with a predefined set of networks comprising the Default mode, SensoriMotor, Visual, Salience, Dorsal attention, FrontoParietal, Language and Cerebellar network. Independent components which were clearly identified with a network were considered for further analysis and used for a seed based connectivity analysis.

For second level analysis, a weighted General Linear Model (GLM) approach was used to make inferences about differences in functional connectivity between conditions either on a ROI to ROI basis or on seed basis using a data driven approach for seed definition.

### Results and discussion

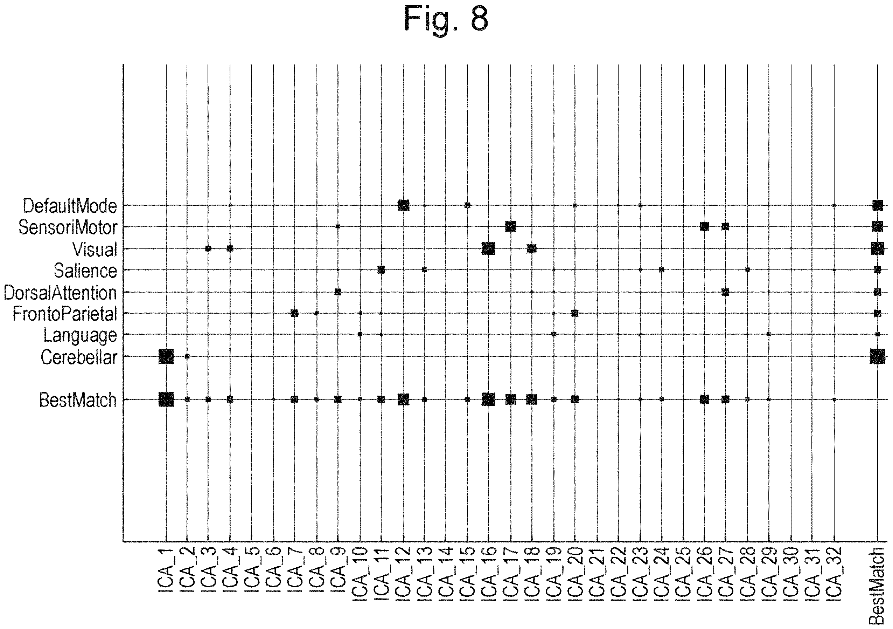

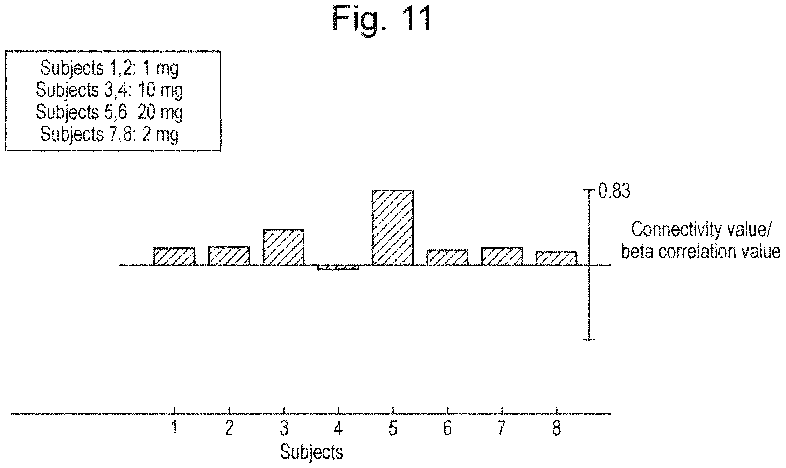

The 32 independent components resulting from the group-ICA were spatially matched with a set of spatially predefined networks including the Default mode (DMN) , SensoriMotor, Visual, Salience, Dorsal attention, FrontoParietal, Language and Cerebellar networks. Only the IC12 (DMN), IC16 and IC18 (Visual network), IC17, IC26 and IC27 (SensoriMotor network) and IC1 (Cerebellar network) could clearly be identified with a network and were considered as a seed regions for a seed based connectivity analysis (see Figure 8). This analysis revealed a significant increase in connectivity after dosing for IC12 (DMN) and IC18 (visual network) when comparing baseline conditions with all dosing conditions. This effect was observed for all four individuals at the key doses of 1 and 2 mg (see Figure 11). When using bivariate correlation measures to determine connectivity, the resulting beta correlation values will represent functional connectivity between a seed and target area (separately for each subject and condition) in terms of Fisher transformed correlation coefficients. In essence, the beta correlation values represent the strength of the functional connectivity between two regions. These values are unitless. These beta correlation values are shown in the Y-axis of Figure 11.

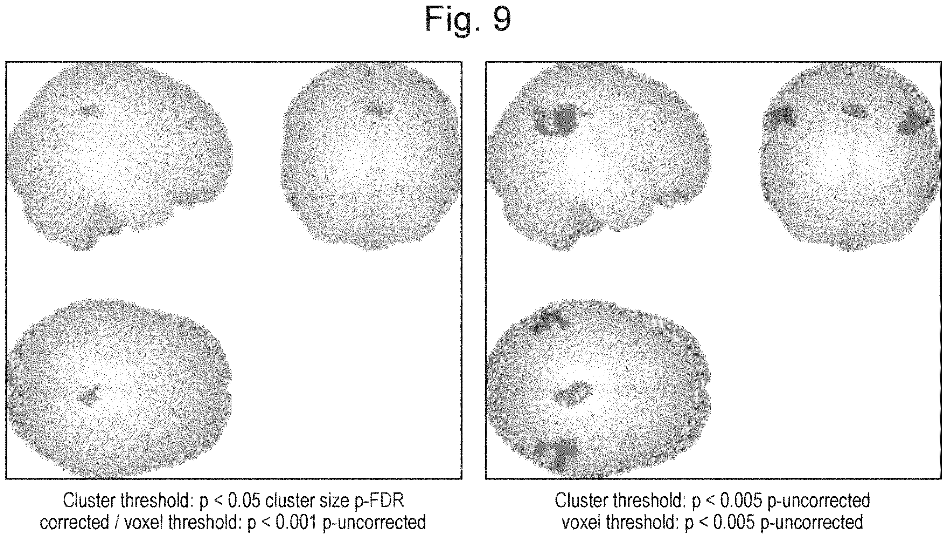

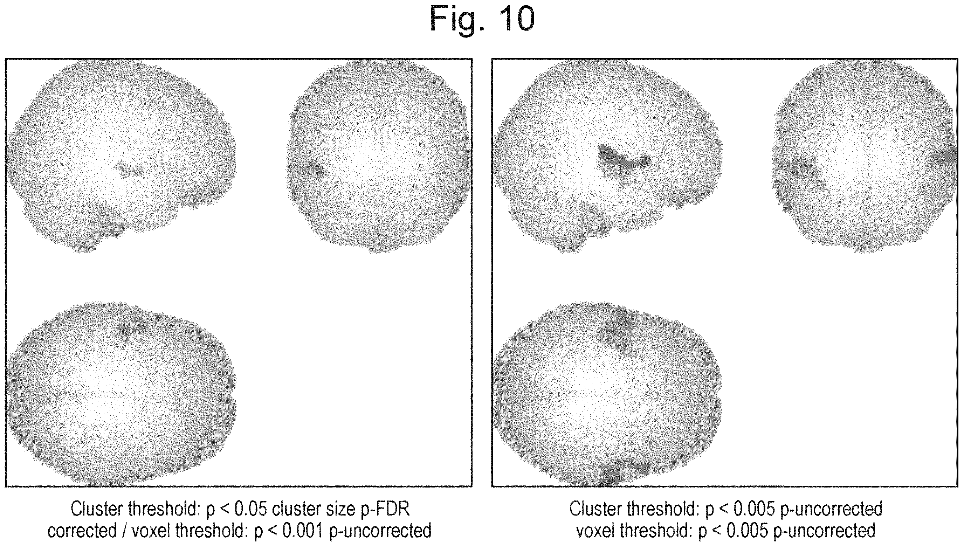

Figure 9 shows that for IC12, connectivity within the DMN was increased. Loss of activity in the DMN occurs during Alzheimer's disease (see *"*The significance of the Default Mode Network (DMN) in Neurological and Neuropsychiatric Disorders: A Review ", Mohan et al., YJBM 89 2016 pp 49-57 and *"*Default Mode Network Complexity and Cognitive Decline in Mild Alzheimer's Disease" Grieder et al., Frontiers in Neuroscience 2018, vol 12, Article 770). Therefore, improvements in connectivity in this region are a promising result and indicate that the compound has potential in treating disorders associated with cognitive decline. The left and right figures in both Figures 9 and 10 are both post dose, but applying different significance level cut offs (the left hand is corrected for false discovery rate). The colours show areas that show an increase in synchronous changes in BOLD signals changes, indicating an increase in functional connectivity. Similar analyses for assessing DMN connectivity may be found in Schmidt et al., "Default Mode, Dorsal Attention and Auditory Resting State Networks Exhibit Differential Functional Connectivity in Tinnitus and Hearing Loss", PLoS ONE 8(10): e76488. https://doi.org/10.1371/journal.pone.0076488. A similar subject-by-subject analysis to that shown in Figure 11 below is shown in Figure S1 in the supplementary information of that paper.

Figure 10 shows that connectivity between IC18 (Visual network) and auditory network was increased. This provides further evidence of an increase in brain connectivity with doses of the compound of example 1 at relatively low occupancy levels.

Increases in DMN connectivity are viewed as a 'proxy' for improved cognition. Mild cognitive impairment and Alzheimer's disease are linked to decreased DMN functional connectivity (see Hafkemeijer et al "Imaging the default mode network in aging and dementia", Biochimica et Biophysica Acta, 1822, 2012, 431-441). Indeed, changes in DMN connectivity are also associated with deficits in cognition that arise naturally with advancing age (see Sambataro et al., "Age-related alterations in default mode network: imipact on working memory performance", Neurobiol Aging, 2010, 31(5), 839-852). Changes in DMN connectivity are also associated with schizophrenia, and drugs used to treat schizophrenia such as olanzapine have been found to increase DMN connectivity in certain regions (see Sambataro et al., "Treatment with Olanzapine is Associated with Modulation of the Default Mode Network in Patients with Schizophrenia", Neuropsychopharmacology 2010, 35, 904-912). Therefore, the ability of the compound of example 1 to modulate and enhance connectivity in the DMN at low doses is indicative of its utility in improving cognition and treating disorders of this nature.

The following are additional aspects of the present invention.
1. A compound which is a substituted 2-oxo-1-pyrrolidinyl triazole of formula (I) 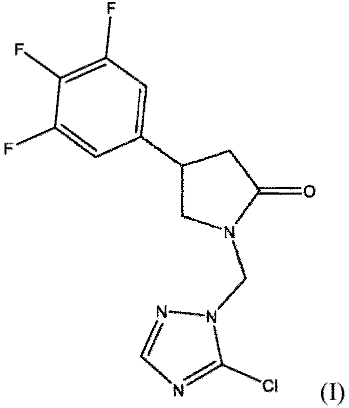 or an isomer or a pharmaceutically acceptable salt thereof, for use in the treatment of a cognitive disorder in a subject, wherein the compound is administered to the subject in an amount of from 0.2 to 5 mg.
2. The compound for use according to 1, wherein the compound is administered to the subject in an amount of from 0.3 to 4 mg.
3. The compound for use according to 1 or 2, wherein the compound is administered to the subject in an amount of from 1 to 3 mg.
4. The compound for use according to any of 1 to 3, wherein the compound is administered to the subject as a single unit dosage comprising from 0.2 to 5 mg of the compound,
   preferably as a single unit dosage comprising from 0.3 to 4 mg of the compound,
   more preferably as a single unit dosage comprising from 1 to 3 mg of the compound.
5. A compound which is a substituted 2-oxo-1-pyrrolidinyl triazole of formula (I) 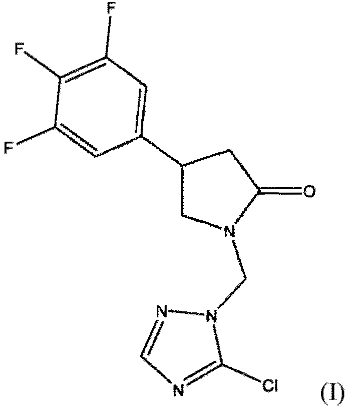 or an isomer or a pharmaceutically acceptable salt thereof, for use in the treatment of a cognitive disorder in a subject, wherein the compound is administered to the subject in an amount that provides an synaptic vesicle glycoprotein 2A (SV2A) occupancy of from 10% to 80% at trough level after once daily dosing for at least 10 days.
6. The compound for use according to 5, wherein the compound is administered to the subject in an amount that provides an SV2A occupancy of from 10% to 70% at trough level after once daily dosing for at least 10 days.
7. The compound for use according to 5 or 6, wherein the compound is administered to the subject in an amount that provides an SV2A occupancy of from 20% to 50% at trough level after once daily dosing for at least 10 days.
8. The compound for use according to any one of 5 to 7, wherein the compound is administered to the subject in an amount as defined in any one of 1 to 4.
9. A pharmaceutical composition comprising a compound which is a substituted 2-oxo-1-pyrrolidinyl triazole of formula (I) 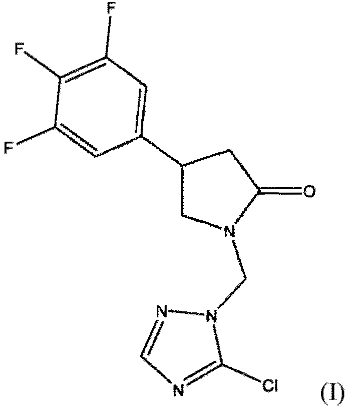 or an isomer or a pharmaceutically acceptable salt thereof, and one or more pharmaceutically acceptable excipients, for use in the treatment of a cognitive disorder in a subject, wherein the pharmaceutical composition is administered to deliver to the subject an amount of the compound as defined in any one of 1 to 8.
10. The compound or pharmaceutical composition for use according to any one of 1 to 9, wherein the compound is (4R)-1-[(5-chloro-1H-1,2,4-triazol-1-yl)methyl]-4-(3,4,5- trifluorophenyl)pyrrolidin-2-one, or a pharmaceutically acceptable salt thereof.
11. The compound or pharmaceutical composition for use according to any one of 1 to 10, wherein the compound is in the free base form.
12. The compound or pharmaceutical composition for use according to any one of 1 to 11, wherein the compound or pharmaceutical composition is for use in enhancing or improving cognitive ability or counteracting cognitive decline.
13. The compound or pharmaceutical composition for use according to any one of 1 to 12, wherein the cognitive disorder is selected from autism, dyslexia, attention deficit hyperactivity disorder, obsessive compulsive disorders, psychosis, bipolar disorders, depression (major depressive disorder), Tourette's syndrome and disorders of learning in children, adolescents and adults, Age Associated Memory Impairment, Age Associated Cognitive Decline, Parkinson's Disease, Down's Syndrome, traumatic brain injury, Huntington's Disease, Progressive Supranuclear Palsy (PSP), HIV infection, stroke, vascular diseases, Pick's or Creutzfeldt-Jacob diseases, multiple sclerosis (MS), other white matter disorders and drug-induced cognitive worsening, Alzheimer's disease, schizophrenia, Lewy-bodies disease, front-temporal lobe degeneration, vascular narrowing or blockage in the brain (i.e. vascular dementia also known as multi-infarct dementia), head trauma, subjective cognitive decline and mild cognitive impairment,
   preferably wherein the cognitive disorder is selected from subjective cognitive decline, Age Associated Memory Impairment, mild cognitive impairment, Alzheimer's disease, cognitive impairment in major depressive disorder and cognitive impairment in a subject with remitted depression following multiple episodes of major depressive disorder.
14. The compound or pharmaceutical composition for use according to any one of 1 to 13, wherein the compound or pharmaceutical composition is administered orally.
15. The compound or pharmaceutical composition for use according to any one of 1 to 14, wherein the amount of the compound as defined in any one of 1 to 4 is administered to the subject daily.

## Claims

1. A compound which is a substituted 2-oxo-1-pyrrolidinyl triazole of formula (I) 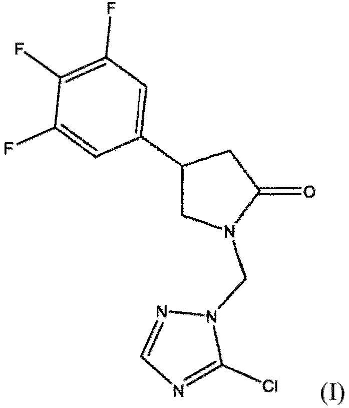 or a pharmaceutically acceptable salt thereof, for use in the treatment of a cognitive disorder in a subject, wherein the compound is administered to the subject in an amount of from 0.2 to 5 mg.

2. The compound for use according to claim 1, wherein the compound is administered to the subject in an amount of from 0.3 to 4 mg.

3. The compound for use according to claim 1 or claim 2, wherein the compound is administered to the subject in an amount of from 1 to 3 mg.

4. The compound for use according to any preceding claim, wherein the compound is administered to the subject as a single unit dosage comprising from 0.2 to 5 mg of the compound,
preferably as a single unit dosage comprising from 0.3 to 4 mg of the compound,
more preferably as a single unit dosage comprising from 1 to 3 mg of the compound.

5. A pharmaceutical composition comprising a compound which is a substituted 2-oxo-1-pyrrolidinyl triazole of formula (I) 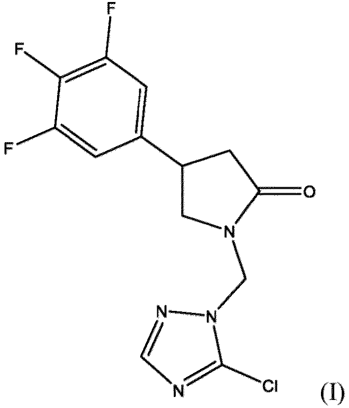 or a pharmaceutically acceptable salt thereof, and one or more pharmaceutically acceptable excipients, for use in the treatment of a cognitive disorder in a subject, wherein the pharmaceutical composition is administered to deliver to the subject an amount of the compound as defined in any one of claims 1 to 4.

6. The compound or pharmaceutical composition for use according to any one of the preceding claims, wherein the compound comprises at least 90% (4R)-1-[(5-chloro-1H-1,2,4-triazol-1-yl)methyl]-4-(3,4,5-trifluorophenyl)pyrrolidin-2-one or a pharmaceutically acceptable salt thereof.

7. The compound or pharmaceutical composition for use according to any one of the preceding claims, wherein the compound comprises at least 95% (4R)-1-[(5-chloro-1H-1,2,4-triazol-1-yl)methyl]-4-(3,4,5- trifluorophenyl)pyrrolidin-2-one or a pharmaceutically acceptable salt thereof.

8. The compound or pharmaceutical composition for use according to any one of the preceding claims, wherein the compound comprises at least 99% (4R)-1-[(5-chloro-1H-1,2,4-triazol-1-yl)methyl]-4-(3,4,5- trifluorophenyl)pyrrolidin-2-one or a pharmaceutically acceptable salt thereof.

9. The compound or pharmaceutical composition for use according to any one of the preceding claims, wherein the compound is (4R)-1-[(5-chloro-1H-1,2,4-triazol-1-yl)methyl]-4-(3,4,5- trifluorophenyl)pyrrolidin-2-one, or a pharmaceutically acceptable salt thereof.

10. The compound or pharmaceutical composition for use according to any one of the preceding claims, wherein the compound is in the free base form.

11. The compound or pharmaceutical composition for use according to any one of the preceding claims, wherein the compound or pharmaceutical composition is for use in enhancing or improving cognitive ability or counteracting cognitive decline.

12. The compound or pharmaceutical composition for use according to any one of the preceding claims, wherein the cognitive disorder is selected from autism, dyslexia, attention deficit hyperactivity disorder, obsessive compulsive disorders, psychosis, bipolar disorders, depression (major depressive disorder), Tourette's syndrome and disorders of learning in children, adolescents and adults, Age Associated Memory Impairment, Age Associated Cognitive Decline, Parkinson's Disease, Down's Syndrome, traumatic brain injury, Huntington's Disease, Progressive Supranuclear Palsy (PSP), HIV infection, stroke, vascular diseases, Pick's or Creutzfeldt-Jacob diseases, multiple sclerosis (MS), other white matter disorders and drug-induced cognitive worsening, Alzheimer's disease, schizophrenia, Lewy-bodies disease, front-temporal lobe degeneration, vascular narrowing or blockage in the brain (i.e. vascular dementia also known as multi-infarct dementia), head trauma, subjective cognitive decline and mild cognitive impairment.

13. The compound or pharmaceutical composition for use according to claim 12, wherein the cognitive disorder is selected from subjective cognitive decline, Age Associated Memory Impairment, mild cognitive impairment, Alzheimer's disease, cognitive impairment in major depressive disorder and cognitive impairment in a subject with remitted depression following multiple episodes of major depressive disorder.

14. The compound or pharmaceutical composition for use according to any one of the preceding claims, wherein the compound or pharmaceutical composition is administered orally.

15. The compound or pharmaceutical composition for use according to any one of the preceding claims, wherein the amount of the compound as defined in any one of claims 1 to 4 is administered to the subject daily.
